# EUROPEAN PATENT SPECIFICATION

(11) **EP 3 266 792 B1**
(45) Date of publication and mention of the grant of the patent: **30.12.2020**
(21) Application number: 16758925.8
(22) Date of filing: 02.03.2016
(51) Int. Cl.: C07K 1/10, C07C 43/225, C07C 43/23, C07C 217/58, C07K 1/30, C07K 1/02

(54) **PEPTIDE SYNTHESIS METHOD**
PEPTIDSYNTHESEVERFAHREN
PROCÉDÉ DE SYNTHÈSE PEPTIDIQUE

(30) Priority: 04.03.2015 JP 2015042651
(43) Date of publication of application: 10.01.2018
(73) Proprietor: Jitsubo Co., Ltd., Yokohama-shi, Kanagawa 230-0045 (JP)
(72) Inventor: KONO, Yusuke, Yokohama-shi, Kanagawa 2300045 (JP); SUZUKI, Hideaki, Yokohama-shi, Kanagawa 2300045 (JP); MUTO,Susumu, Yokohama-shi, Kanagawa 2300045 (JP)
(74) Representative: Huenges, Martin
(86) International application number: PCT/JP2016/056319
(87) International publication number: WO 2016/140232

(56) References cited:
- EP-A1- 2 716 650
- WO-A1-2006/104166
- WO-A1-2007/034812
- WO-A1-2007/099656
- WO-A1-2007/122847
- JP-A- H06 509 821
- JP-A- 2003 183 298
- JP-A- 2003 183 298
- JP-A- 2003 500 415
- JP-A- 2004 059 509
- CARPINO, L.A. ET AL.: 'Rapid, Continuous Solution-Phase PeptideSynthesis: Application to Peptides of Pharmaceutical Interest' ORG. PROCESS RES. DEV. vol. 7, no. 1, 2003, pages 28 - 37, XP055008431
- EGGEN, I.F. ET AL.: 'A novel method for repetitive peptide synthesis in solution without isolation of intermediates' J. PEPT. SCI. vol. 11, no. 10, 2005, pages 633 - 641, XP002420466
- Daisuke Takahashi ET AL: "Novel diphenylmethyl-Derived Amide Protecting Group for Efficient Liquid-Phase Peptide Synthesis: AJIPHASE", ORGANIC LETTERS, vol. 14, no. 17, 7 September 2012 (2012-09-07), pages 4514-4517, XP55246787, ISSN: 1523-7060, DOI: 10.1021/ol302002g
- DAISUKE TAKAHASHI ET AL: "Development of an efficient liquid-phase peptide synthesis protocol using a novel fluorene-derived anchor support compound with Fmoc chemistry; AJIPHASE", TETRAHEDRON LETTERS, ELSEVIER LTD, AMSTERDAM, NL, vol. 53, no. 15, 2 February 2012 (2012-02-02), pages 1936-1939, XP028466059, ISSN: 0040-4039, DOI: 10.1016/J.TETLET.2012.02.006 [retrieved on 2012-02-09]
- Daisuke Takahashi ET AL: "Supplementary Data Development of an efficient liquid-phase peptide synthesis protocol using a novel fluorine-derived anchor support compound with Fmoc chemistry; AJIPHASE ", tetrahedron letters, vol. 53, 9 February 2012 (2012-02-09), pages s1-s5, XP055661328,

## Description

### [Technical Field]

The present invention relates to a novel peptide synthesis method, specifically, to a peptide synthesis method capable of easily performing an accurate peptide elongation reaction.

### [Background Art]

Conventionally, methods in which specific components dissolved in a liquid are separated as a solid, in a chemical process, are widely used. The reason for this is that separation and purification after the reaction become easy by solidifying (crystallizing) only specific components. According to this method, for example, a necessary or unnecessary compound is solidified (crystallized) after completion of each reaction in sequential multi-step synthesis such as synthesis of a compound library used in recent developments and studies of pharmaceutical preparations, resultantly, the solidified (crystallized) substance can be easily separated and purified. Thus, process complexity as the conventional problem can be solved.

There is also used a method in which a specific component dissolved is selectively dissolved in a specific phase (selective distribution) in liquid phase separation, to realize separation from other components. This method can contribute to rapid and simplified processes, since a specific component can be separated without solidification (crystallization).

Solidification (crystallization) of a specific component dissolved in a solution or selective dissolution of a specific component into a specific liquid phase (selective distribution) as described above is realized by satisfying certain conditions regarding chemical nature of a compound, physical properties thereof and the solvent. However, conditions of solidification (crystallization) and selective dissolution (selective distribution) should be empirically searched via trial and error in many cases. Particularly in sequential multi-step synthesis, significant cost and time are necessary for process development, since it is necessary to investigate conditions of respective steps based on natures specific to compounds synthesized in respective steps.

Then, a carrier molecule having a linker, which sensitively detects a change of the solvent composition thereby causing a reversible change between the dissolved state and the insolubilized (crystallized) state or causes selective dissolution of a specific component dissolved into a specific phase at high concentration (selective distribution) in liquid phase separation, is suggested. Various compounds can be bonded to such a carrier molecule via a linker. Thus, the bonded compounds can easily undergo a state variation from the dissolved state to the insolubilized (crystallized) state or inversely, together with the carrier molecule. Alternatively, the compounds bonded to the carrier molecule can be dissolved at high concentration selectively (selective distribution) into a specific phase of a liquid separated into several phases.

Further, such a carrier molecule can reversibly repeat the dissolved state and the insolubilized (crystallized) state under approximately the same condition even if the chemical structure of the bonded compound varies by the sequential chemical reaction, or can cause selective dissolution at high concentration (selective distribution) into a specific phase of a liquid separated in several phases. When a carrier molecule which causes a reversible change between the dissolved state and the insolubilized (crystallized) state or can induce the selective distribution state as described above is used, a compound as the separation target can be selectively separated from the uniform dissolved state, using knowledge of general liquid phase reactions in organic chemistry as it is. That is, a specific compound can be separated while leaving other soluble components in the solution after the liquid phase reaction.

As the carrier capable of reversibly repeating the dissolved state and the insolubilized state, various compounds are suggested. For example, a benzyl alcohol compound to which a long chain fatty acid is introduced is suggested by the present inventors (Patent documents 1 to 4), or a fluorene compound to which a long chain fatty acid is introduced (Patent document 5) and a diphenylmethane compound to which a long chain fatty acid is introduced (Patent document 6) are listed.

The peptide synthesis technology includes a solid phase peptide synthesis method (SPPS method) and a liquid phase peptide synthesis method (LPPS method). In the solid phase peptide synthesis method, purification cannot be conducted at each step of the amino acid elongation reaction, in principle. Since the synthesis cost is high, this method is suitable for small volume production. In contrast, the liquid phase peptide synthesis method is widely used for large scale production, however, when the length of the peptide chain increases, the peptide elongation reaction becomes difficult, thus, synthesis of a long chain peptide is problematic.

For this reason, it is suggested to conduct peptide synthesis using the above-described carrier capable of reversibly repeating the dissolved state and the insolubilized state (Patent documents 1 to 6).

Peptide synthesis has a problem of deletion of an amino acid residue occurring in the peptide elongation reaction, and it is problematic also in the case of use of the above-described carrier. For solving deletion of an amino acid residue, equivalents of an amino acid and a condensing agent are increased. However, when an excessive amino acid active species remains in the reaction liquid as its result, there is a new problem that double hit occurs in de-protection of an N-terminal. By this, the yield of the intended amino acid lowers. The double hit denotes that one extra amino acid residue is further inserted into the target peptide.

As the solution means for this, an amino acid active species is removed from the reaction system by washing before de-protection. For example, a method as described below is suggested. There is a suggestion on a method of adding an amine having an anion component such as β-alanine-OBz as a scavenger, for removing an active ester of an amino acid remaining in the reaction system in a liquid phase peptide synthesis method using a benzyloxycarbonyl group (Cbz or Z) or a t-butyloxycarbonyl group (Boc) as a protective group (Patent document 7). In this method, also an anion component protective group of a scavenged body is eliminated simultaneously in de-protecting a protective group from an N-terminal, resulting in the scavenged body becoming water-soluble. Thereafter, liquid-liquid extraction is conducted using an alkaline water-organic solvent, then, a condensing agent and an amino acid component are removed by water. However, when Cbz is used as a protective group, the de-protection catalyst is deactivated if Met or Cys is present on the peptide sequence, that is, this is not applicable. Further, in this method, fluorenylmethoxycarbonyl (Fmoc) cannot be used as a protective group, since dibenzofulvene cannot be removed.

Further, there is a suggestion on a method of removing an amino acid component by liquid-liquid extraction while hydrolyzing an active ester with alkali water (for example, a 5% sodium carbonate aqueous solution), for removing an active ester of an amino acid remaining in the reaction system in a liquid phase peptide synthesis method using Boc as a protective group (Patent document 8). In this method, however, epimerization possibly occurs under strong alkali condition of pH = 11 or more. Additionally, fluorenylmethoxycarbonyl (Fmoc) cannot be used as a protective group, since dibenzofulvene cannot be removed.

Also in peptide synthesis using a carrier capable of reversibly repeating the dissolved state and the insolubilized state described above, an amino acid active species can be removed from the reaction system by washing before de-protection for solving the problem of double hit due to an excessive amino acid active species. However, an amino acid active species cannot be removed completely by washing in some cases by a crystallization operation for insolubilizing the carrier, and there is a problem that twice washing operations after the condensation reaction and after de-protection are required and a large amount of a washing solvent is necessary.

Patent document 9 and Non-Patent documents 2 and 3 describe peptide synthesis methods using condensation wherein a precipitation step takes place between the condensation step and the deprotection step.

Further, there is a report on a method in which Fmoc is de-protected from an N-terminal, then, thiolcarboxylic acid is allowed to coexist and dibenzofulvene is generated, then, liquid-liquid extraction is performed with an alkaline water-organic solvent, then, a condensing agent, an amino acid component and dibenzofulvene are removed by water, thereby removing an amino acid active ester, as the method of removing a Fmoc group in peptide synthesis (Patent document 8). Further, it is disclosed that an active ester may be removed by thiol silica or decomposed with alkali water before de-protection of Fmoc and it is suggested that an active ester of an amino acid is removed in the form of a water-soluble product.

In addition, there is a report on a method in which Fmoc is de-protected from an N-terminal, then, alkyl thiol or solid phase thiol is allowed to coexist and dibenzofulvene is generated, then, condensation is performed, then, a peptide component is crystallized with an ether and solid-liquid separation is conducted, to remove dibenzofulvene adduct, as the method of removing a Fmoc group in peptide synthesis, however, removal of an amino acid active ester is not described (Non-Patent document 1).

### [Citation List]

### [Patent Literature]

Patent Literature 1: Japanese Unexamined Patent Application Publication No. 2 003-183298
Patent Literature 2: Japanese Unexamined Patent Application Publication No. 2 004-059509
Patent Literature 3: WO 2007/034812
Patent Literature 4: WO 2007/122847
Patent Literature 5: WO 2010/104169
Patent Literature 6: WO 2010/113939
Patent Literature 7: Japanese Unexamined Patent Application Publication No. 2003-55396
Patent Literature 8: WO 2007/099656
Patent Literature 9: EP 2 716 650 A1

### [Non-Patent Literature]

Non-Patent Literature 1: James E. Sheppeck II et al., Tetrahedron Letters 41 (2000)
Non-Patent Literature 2: Daisuke Takahashi et al., Organic Letters 14:4514 (2012)
Non-Patent Literature 3: Daisuke Takahashi et al., Tetrahedron Letters 53:1936 (2012)

### [Summary of the Invention]

### [Technical Problem]

The present invention has an object of providing a peptide synthesis method using a carrier capable of reversibly repeating the dissolved state and the insolubilized state, wherein the problem of an amino acid active species existing in the reaction system in de-protection reaction can be easily solved.

### [Solution to Problem]

The present inventors have intensively studied in consideration of the above-described problem and resultantly found that double hit of an amino acid can be prevented in de-protection by deactivating an amino acid active species by a specific scavenger before the de-protection reaction, even if the amino acid active species is not removed from the reaction system before de-protection of an N-terminal, leading to completion of the present invention. The present invention includes the following.
(1) A peptide synthesis method comprising the following steps a to d:
   a. a step of condensing an amino acid having an N-terminal protected with fluorenylmethoxycarbonyl (N-Fmoc protected) or an N-Fmoc protected peptide with an amino acid having a C-terminal protected with a carrier, the carrier being capable to crystallize in response to a change of a composition of a dissolving solvent (C-carrier protected), a C-carrier protected peptide or a C-carrier protected amino acid amide, in the presence of a condensing agent, to obtain an N-Fmoc-C-carrier protected peptide,
   b. a step of subsequently adding to the reaction system resulting from step (a) at least one amine selected from the group consisting of an alkylamine having 1 to 14 carbon atoms, an aromatic amine having 1 to 14 carbon atoms and hydroxyl amine, wherein the alkylamine or the aromatic amine is a primary or secondary amine,
   c. thereafter a step of de-protecting the N-terminal, and
   d. a step of changing the composition of the solvent dissolving the resultant C-carrier protected peptide, to crystallize and separate the C-carrier protected peptide.
(2) The peptide synthesis method according to the above (1), wherein said carrier is a compound selected from the group consisting of
   a compound having the following structure (in the present specification, referred to as "Ka" in some cases): 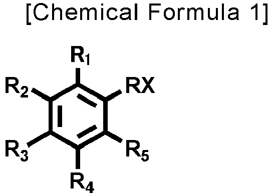 wherein R₁ and R₅ represent a hydrogen atom, and R₂, R₃ and R₄ represent an alkoxyl group having 8 to 30 carbon atoms, preferably an alkoxyl group having 11 to 22 carbon atoms. In the formula, RX is a group represented by the following formula 2 and binding to the C-terminal of a peptide or an amino acid, 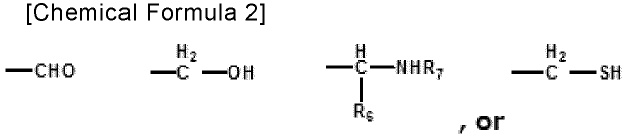 wherein R₇ represents a hydrogen atom, an alkyl group having 1 to 6 carbon atoms, a benzyl group or an alkoxy-substituted benzyl group, and R₆ represents a hydrogen atom, a phenyl group or an alkoxy-substituted phenyl group. The above-described formula is shown in the state before binding to the C-terminal of a peptide or an amino acid;
   a compound having the following structure (in the present specification, referred to as "Kb" in some cases): 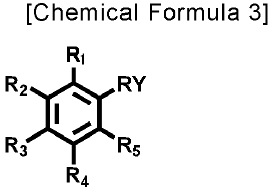 wherein R₂, R₄ and R₅ represent a hydrogen atom, and R₁ and R₃ represent an alkoxyl group having 12 to 30 carbon atoms, preferably an alkoxyl group having 18 to 22 carbon atoms. In the formula, RY is a group represented by the following formula 4 and binding to the C-terminal of a peptide or an amino acid, 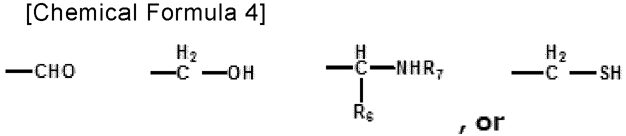 wherein R₇ represents a hydrogen atom, an alkyl group having 1 to 6 carbon atoms, a benzyl group or an alkoxy-substituted benzyl group, and R₆ represents a hydrogen atom, a phenyl group or an alkoxy-substituted phenyl group. The above-described formula is shown in the state before binding to the C-terminal of a peptide or an amino acid; and
   a compound having the following structure (in the present specification, referred to as "Kc" in some cases): 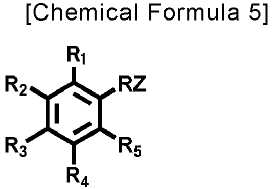 wherein R₁ , R₃ and R₅ represent a hydrogen atom, and R₂ and R₄ represent an alkoxyl group having 12 to 30 carbon atoms, preferably an alkoxyl group having 18 to 22 carbon atoms. In the formula, RZ is a group represented by the following formula 6 and binding to the C-terminal of a peptide or an amino acid, 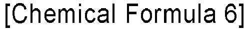 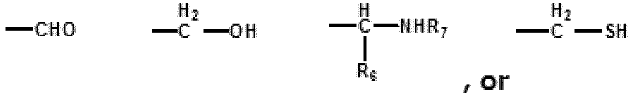 wherein R₇ represents a hydrogen atom, an alkyl group having 1 to 6 carbon atoms, a benzyl group or an alkoxy-substituted benzyl group, and R₆ represents a hydrogen atom, a phenyl group or an alkoxy-substituted phenyl group. The above-described formula is shown in the state before binding to the C-terminal of a peptide or an amino acid.
(3) The peptide synthesis method according to the above (1), wherein said carrier is a compound selected from the group consisting of 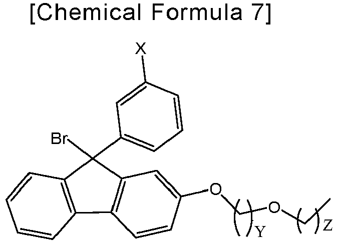 wherein, X represents a halogen, Y is an integer of 8 to 12 and Z is an integer of 17 to 29, 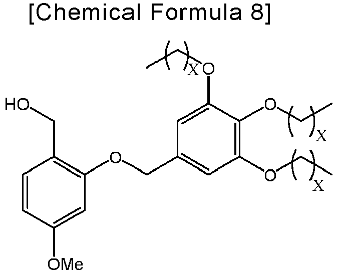 wherein X each independently represent an integer of 7 to 21, and 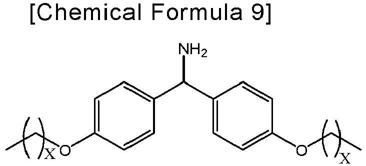 wherein X each independently represent an integer of 11 to 29.
   The above-described formulae are shown in the state before binding to the C-terminal of a peptide or an amino acid.
(4) The peptide synthesis method according to the above (1), wherein said carrier is a compound selected from the group consisting of 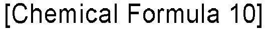 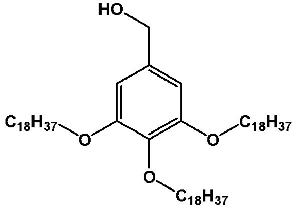 (3,4,5-trioctadecylbenzyl alcohol) 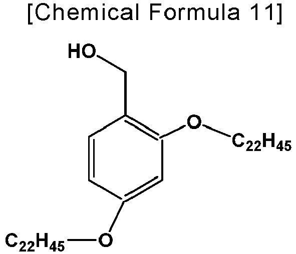 (2,4-didocosyloxybenzyl alcohol) 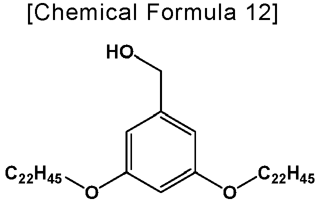 (3,5-didocosyloxybenzyl alcohol) 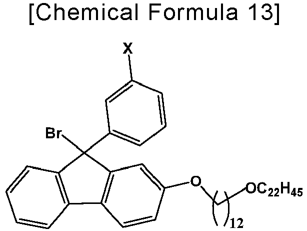 wherein X is F or CI 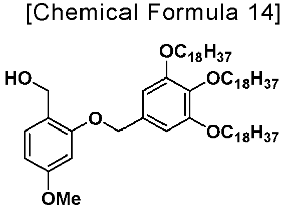 ((2-(3,4,5-trioctadecyloxybenzyl)-4-methoxybenzyl alcohol)
   , and 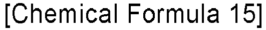 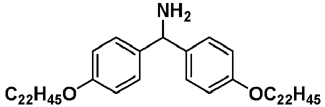 (Bis-(4-docosyloxyphenyl)-methylamine).
   The above-described formulae are shown in the state before binding to the C-terminal of a peptide or an amino acid.
(5) The peptide synthesis method according to any one of the above (1) to (4), wherein said amine is an alkylamine having 1 to 10 carbon atoms or hydroxylamine.
(6) The peptide synthesis method according to any one of the above (1) to (5), wherein said amine is an alkylamine having 3 or 4 carbon atoms.
(7) The peptide synthesis method according to any one of the above (1) to (6), wherein the amine equivalent in the step b is 1 to 30-fold amount with respect to the amino acid equivalent theoretically remaining after the condensation reaction of the step a.
(8) The peptide synthesis method according to any one of the above (1) to (7), wherein the composition changing means for changing the composition of the solvent dissolving the resultant C-carrier protected peptide is performed by concentrating the solvent of the solution, then, adding a poor solvent to attain solidification.
(9) The peptide synthesis method according to the above (8), wherein said poor solvent is a solvent selected from the group consisting of acetonitrile, aqueous acetonitrile, methanol, aqueous methanol and water.
(10) The peptide synthesis method according to any one of the above (1) to (9), comprising conducting repetition of the step a to the step d using the C-carrier protected peptide crystallized and separated in the step d.
(11) The peptide synthesis method according to any one of the above (1) to (10), further comprising the following step:
   e. a step of washing the crystal of the C-carrier protected peptide crystallized and separated with an organic solvent.
(12) The peptide synthesis method according to any one of the above (1) to (11), wherein said steps a to c are conducted in one pot synthesis.

### [Advantageous Effect of Invention]

The peptide synthesis method of the present invention can solve the problem of an amino acid active species existing in the reaction system in the de-protection reaction by a simple means and is excellent in versatility. The peptide synthesized by the present invention has less problems of deletion of an amino acid and double hit, thus, according to the method of the present invention, a peptide of high quality can be synthesized at high yield.

### [Description of Embodiments]

Hereinafter, the present invention will be illustrated and described in detail with reference to the exemplary embodiments, along with the preferred methods and materials which can be used in practice of the present invention.

Unless otherwise specified in the sentences, any technical terms and scientific terms used in the present specification have the same meaning as those generally understood by those of ordinary skill in the art to which the present invention belongs.

Terms used in the present specification will be explained below.

### 1. N-Fmoc protected amino acid and peptide

The amino acid having an N-terminal protected by fluorenylmethoxycarbonyl (Fmoc) (N-Fmoc protected amino acid) denotes an amino acid in which an amino group of the amino acid is protected by Fmoc, while a carbonyl group is not protected and is reactive.

The peptide having an N-terminal protected by fluorenylmethoxycarbonyl (Fmoc) (N-Fmoc protected peptide) denotes a peptide in which an amino group at the N-terminal of the peptide chain is protected by Fmoc, while a carbonyl group at the C-terminal is not protected and is reactive.

When the N-Fmoc protected amino acid or the N-Fmoc protected peptide has a functional group rich in reactivity such as a hydroxyl group, an amino group, a guanidyl group, a carboxyl group, or a sulfhydryl group, a general protective group used in peptide chemistry may be introduced into these functional groups, and after completion of the reaction the protective group is removed if necessary, thus, the target compound can be obtained.

The protective group for a hydroxyl group includes a tBu group, a Trt group, a Bz group, an acetyl group, and a silyl group, the protective group for an amino group includes a Boc group, a Fmoc group, a Cbz group, a Trt group, an Mmt group, and an ivDde group, the protective group for a guanidyl group includes a Pbf group, a Pmc group, and a nitro group, the protective group for a carboxyl group includes a tBu group, a methyl group, an ethyl group, and a Bz group, and the protective group for a sulfhydryl group includes a Trt group, an Acm group, a tBu group, and an S-tBu group.

### 2. C-carrier protected amino acid, peptide and amino acid amide

The amino acid having a C-terminal protected by a carrier which is crystallized according to a change of a composition of a dissolving solvent (C-carrier protected amino acid) denotes an amino acid in which a carboxyl group of the amino acid is protected by a carrier described later, while an amino group is not protected and is reactive.

The peptide having a C-terminal protected by a carrier which is crystallized according to a change of a composition of a dissolving solvent (C-carrier protected peptide) denotes a peptide in which a carboxyl group at the C-terminal of the peptide chain is protected by a carrier described later, while an amino group at the N-terminal is not protected and is reactive.

The amino acid amide having a C-terminal protected by a carrier which is crystallized according to a change of a composition of a dissolving solvent (C-carrier protected amino acid amide) denotes an amino acid amide in which a carboxyl group thereof is protected by a carrier described later, while an amino group is not protected and is reactive.

When the C-carrier protected amino acid or C-carrier protected peptide or C-carrier protected amino acid amide has a functional group rich in reactivity such as a hydroxyl group, an amino group, a guanidyl group, a carboxyl group, or a sulfhydryl group, a general protective group used in peptide chemistry may be introduced into these functional groups, and after completion of the reaction the protective group is removed if necessary, thus, the target compound can be obtained.

The protective group for a hydroxyl group includes a tBu group, a Trt group, a Bz group, an acetyl group, and a silyl group, the protective group for an amino group includes a Boc group, a Fmoc group, a Cbz group, a Trt group, an Mmt group, and an ivDde group, the protective group for a guanidyl group includes a Pbf group, a Pmc group, and a nitro group, the protective group for a carboxyl group includes a tBu group, a methyl group, an ethyl group, and a Bz group, and the protective group for a sulfhydryl group includes a Trt group, an Acm group, a tBu group, and an S-tBu group.

### 3. Carrier which is crystallized according to change of composition of dissolving solvent

The carrier which is crystallized according to a change of a composition of a dissolving solvent denotes a carrier showing a reversible change between the dissolved state and the solidified (crystallized) state according to a change of the composition of the solvent dissolving the carrier. Specific examples thereof include the following compounds, and a C-carrier protected amino acid, a C-carrier protected peptide or a C-carrier protected amino acid amide in which the C-terminal is protected by the carrier, used in the present invention, can be produced by bonding these compounds to the C-terminal of a peptide or an amino acid.

### 3-1. Carrier A

A compound having the following structure (in the present specification, referred to as "Ka" in some cases): 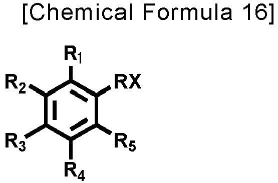 wherein R₁ and R₅ represent a hydrogen atom, and R₂, R₃ and R₄ represent an alkoxyl group having 8 to 30 carbon atoms. In the formula, RX is a group represented by the following formula and bonding to the C-terminal of a peptide or an amino acid, 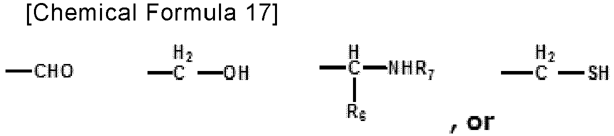 wherein R₇ represents a hydrogen atom, an alkyl group having 1 to 6 carbon atoms, a benzyl group or an alkoxy-substituted benzyl group, and R₆ represents a hydrogen atom, a phenyl group or an alkoxy-substituted phenyl group.

In the above-described formula, R₂, R₃ and R₄ have a number of carbon atoms of more preferably 8 to 22, further preferably 12 to 18.

In the above-described formula, RX is more preferably a hydroxymethyl group, an aminomethyl group or a mercaptomethyl group, further preferably a hydroxymethyl group.

The compound included in the above-described formula is preferably 3,4,5-trioctadecylbenzyl alcohol, 3,4,5-trioctadecylbenzylamine or 3,4,5-trioctadecylben-zylthiol, further preferably 3,4,5-trioctadecylbenzyl alcohol or 3,4,5-trioctadecylbenzylamine, most preferably 3,4,5-trioctadecylbenzyl alcohol represented by the following formula.

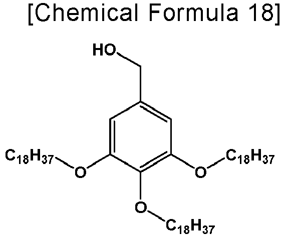

For bonding of the above-described compound to the C-terminal of a peptide or an amino acid, methods generally used in peptide synthesis can be used also in the present invention without restriction, and for example, the bonding can be carried out by esterification using DIPCI.

### 3-2. Carrier B

A compound having the following structure (in the present specification, referred to as "Kb" in some cases): 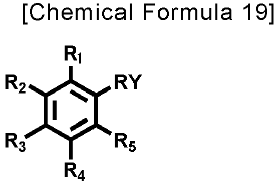 wherein R₂, R₄ and R₅ represent a hydrogen atom, and R₁ and R₃ represent an alkoxyl group having 12 to 30 carbon atoms. In the formula, RY is a group represented by the following formula and bonding to the C-terminal of a peptide or an amino acid, 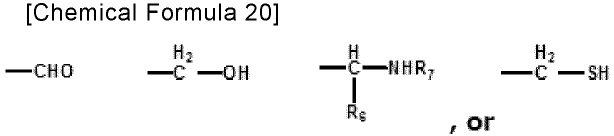 wherein R₇ represents a hydrogen atom, an alkyl group having 1 to 6 carbon atoms, a benzyl group, or an alkoxy-substituted benzyl group, and R₆ represents a hydrogen atom, a phenyl group or an alkoxy-substituted phenyl group.

In the above-described formula, R₁ and R₃ more preferably have a number of carbon atoms of 18 to 22.

In the above-described formula, RX is more preferably a hydroxymethyl group, an aminomethyl group or a mercaptomethyl group, further preferably a hydroxymethyl group.

The compound included in the above-described formula is preferably 2,4-didocosyloxybenzyl alcohol, 2,4-didocosyloxybenzylamine or 2,4-didocosyloxybenzylthiol, further preferably 2,4-didocosyloxybenzyl alcohol or 2,4-didocosyloxybenzylamine, most preferably 2,4-didocosyloxybenzyl alcohol represented by the following formula.

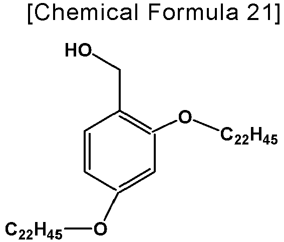

For bonding of the above-described compound to the C-terminal of a peptide or an amino acid, methods generally used in peptide synthesis can be used also in the present invention without restriction, and for example, the bonding can be carried out by esterification using DIPCI.

### 3-3. Carrier C

A compound having the following structure (in the present specification, referred to as "Kc" in some cases): 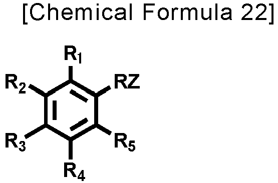 wherein R₁, R₃ and R₅ represent a hydrogen atom, and R₂ and R₄ represent an alkoxyl group having 12 to 30 carbon atoms. In the formula, RZ is a group represented by the following formula and bonding to the C-terminal of a peptide or an amino acid, 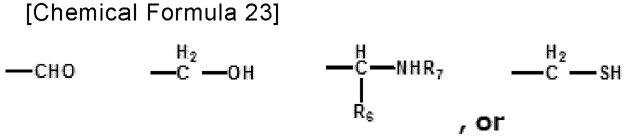 wherein R₇ represents a hydrogen atom, an alkyl group having 1 to 6 carbon atoms, a benzyl group or an alkoxy-substituted benzyl group, and R₆ represents a hydrogen atom, a phenyl group or an alkoxy-substituted phenyl group.

In the above-described formula, R₂ and R₄ more preferably have a number of carbon atoms of 18 to 22.

In the above-described formula, RX is more preferably a hydroxymethyl group, an aminomethyl group or a mercaptomethyl group, further preferably a hydroxymethyl group.

The compound included in the above-described formula is preferably 3,5-didocosyloxybenzyl alcohol, 3,5-didocosyloxybenzylamine or 3,5-didocosyloxybenzylthiol, further preferably 3,5-didocosyloxybenzyl alcohol or 3,5-didocosyloxybenzylamine, most preferably 3,5-didocosyloxybenzyl alcohol represented by the following formula.

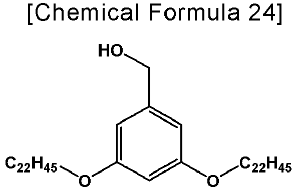

For bonding of the above-described compound to the C-terminal of a peptide or an amino acid, methods generally used in peptide synthesis can be used also in the present invention without restriction, and for example, the bonding can be carried out by esterification using DIPCI.

### 3-4. Carrier D

A compound having the following structure: 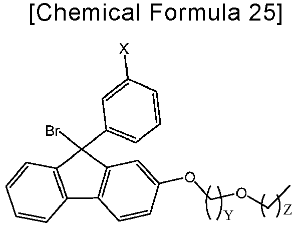 wherein X is a halogen, Y is an integer of 8 to 12 and Z is an integer of 17 to 29.

In the above-described formula, X is preferably F or CI, more preferably F.

A compound represented by the following formula is most preferable. 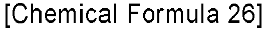 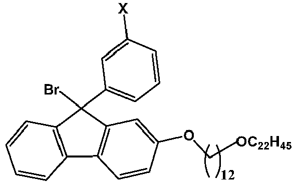 wherein X is F or CI.

For bonding of the above-described compound to the C-terminal of a peptide or an amino acid, methods generally used in peptide synthesis can be used also in the present invention without restriction, and for example, the bonding can be carried out by esterification using a basic catalyst.

### 3-5. Carrier E

A compound having the following structure: 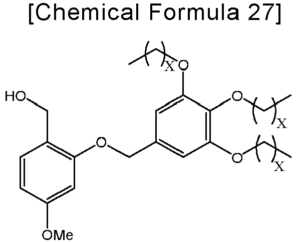 wherein X each independently represent an integer of 7 to 21.

2-(3,4,5-trioctadecyloxybenzyl)-4-methoxybenzyl alcohol represented by the following formula is most preferable.

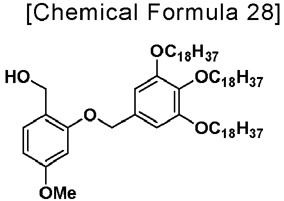

For bonding of the above-described compound to the C-terminal of a peptide or an amino acid, methods generally used in peptide synthesis can be used also in the present invention without restriction, and for example, the bonding can be carried out by esterification using DIPCI.

### 3-6. Carrier F

A compound having the following structure: 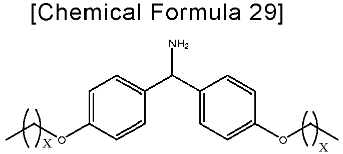 wherein X each independently represent an integer of 11 to 29.

Bis-(4-docosyloxyphenyl)-methylamine represented by the following formula is most preferable.

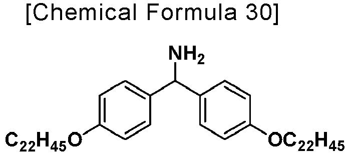

For bonding of the above-described compound to the C-terminal of a peptide or an amino acid, methods generally used in peptide synthesis can be used also in the present invention without restriction, and for example, the bonding can be carried out by amidation using DICPI/HOBt.

### 4. Production method of peptide

The peptide synthesis method of the present invention can be suitably used in a peptide production method using a carrier capable of reversibly repeating the dissolved state and the insolubilized state.

The peptide synthesis method of the present invention comprises the steps as defined in the claims. Some steps as listed here are described below in more detail.
(i) Coupling step
   a step of condensing an N-Fmoc protected amino acid or peptide with a C-carrier protected amino acid or peptide in the presence of a condensing agent, to obtain an N-Fmoc-C-carrier protected peptide,
(ii) a step of forming a scavenged body of an amino acid active ester, using a primary or secondary alkylamine having 1 to 14 carbon atoms or a primary or secondary aromatic amine having 1 to 14 carbon atoms or hydroxylamine (hereinafter, referred to as amine scavenger),
(iii) a step of removing an Fmoc group from the N-terminal of a peptide, to de-protect the N-terminal,
(iv) a step of crystallizing and separating a carrier to which a peptide is bonded (C-carrier protected peptide), using a poor solvent,
(v) a step of repeating the above-described steps (i) to (v) necessary times, and
(vi) a step of removing the carrier from a peptide, to conduct final de-protection.
   Further, after the above-described step (iv),
(vii) a step of washing the crystallized and separated C-carrier protected peptide with an organic solvent,
   can also be added, and by this, removal of impurities including the scavenged body of an amino acid active ester is more complete.

The respective steps will be explained below.

### 4-1. Coupling step

In this step, for example, an N-Fmoc protected amino acid is mixed with a C-carrier protected peptide, a C-carrier protected amino acid or a C-carrier protected amino acid amide and a condensing agent (preferably, a condensing agent and an activating agent) in a solvent, to obtain an N-Fmoc-C-carrier protected peptide in which one amino acid residue is elongated. If an N-Fmoc protected peptide is used instead of the N-Fmoc protected amino acid, an N-Fmoc-C-carrier protected peptide in which amino acid residues are elongated by the number of amino acid residues of the N-Fmoc protected peptide is obtained.

The method and order of addition of respective components are not particularly restricted, and methods usually used in a coupling step in peptide synthesis can be used.

The use amount of an N-Fmoc protected amino acid or an N-Fmoc protected peptide with respect to a C-protected carrier peptide is usually 1.03 to 8 equivalents, preferably 1.01 to 4 equivalents, more preferably 1.05 to 2 equivalents, further preferably 1.1 to 1.5 equivalents with respect to the C-carrier protected peptide. Below this range, an unreacted C-protected peptide tends to remain, and deletion of an amino acid occurs easily. In the peptide synthesis method of the present invention, unreacted amino acid active esters are scavenged (captured) by an amine scavenger to be added later, and they can be removed easily using an organic solvent, after de-protection of the N-terminal of an N-Fmoc-C-carrier protected peptide. Thus, even if a larger amount of an N-Fmoc protected amino acid or N-Fmoc protected peptide is used, the problem of remaining materials does not occur as compared with conventional methods.

As the condensing agent, condensing agents generally used in peptide synthesis can be used without restriction also in the present invention, and examples thereof include 4-(4,6-dimethoxy-1,3,5-triazin-2-yl)-4-methylmorphonium chloride (DMT-MM), O-(benzotriazol-1-yl)-1,1,3,3-tetramethyluroniumhexafluoro phosphate (HBTU), 0-(7-azabenzotriazol-1-yl)-1,1,3,3-tetramethyluroniumhexafluoro phosphate (HATU), O-(6-chlorobenzotriazol-1-yl)-1,1,3,3-tetramethyluroniumhexafluoro phosphate (HBTU (6-CI)), O-(benzotriazol-1-yl)-1,1,3,3-tetramethyluroniumtetrafluoro borate (TBTU), O-(6-chlorobenzotriazol-1-yl)-1,1,3,3-tetramethyluroniumtetrafluoro borate (TCTU), (1-cyano-2-ethoxy-2-oxoethylideneaminooxy)dimethylamino-morpholino-carbeniumhexafluoro phosphate (COMU), diisopropylcarbodiimide (DIPCI), dicyclohexylcarbodiimide (DCC) and 1-ethyl-3-(3-dimethylaminopropyl)carbodiimide hydrochloride (WSC), and preferable is DMT-MM, HBTU, HATU or COMU. The use amount of the condensing agent is usually 0.7 to 1.5 equivalents, preferably 0.85 to 1.0 equivalents with respect to the N-Fmoc protected amino acid.

In the coupling step, an activating agent is preferably added for promoting the reaction and suppressing side reactions such as racemization. The activating agent is a reagent converting an amino acid to the corresponding active ester or symmetric acid anhydride, thereby facilitating formation of a peptide bond (amide bond), by coexistence with a condensing agent. As the activating agent, activating agents generally used in peptide synthesis can be used without restriction also in the present invention, and examples thereof include HOBt, HOCt, HOAt, HOOBt, HOSu, HOPht, HONb, and pentafluorophenol, and preferable are HOBt, HOOBt, HOCt, HOAt, HONb and HOSu. The use amount of the activating agent is usually 0 to 4.0 equivalents, preferably 0.1 to 1.5 equivalents with respect to the N-Fmoc amino acid.

As the solvent used in the coupling step, solvents generally used in peptide synthesis can be used without restriction also in the present invention, and examples thereof include DMF, NMP, ethyl acetate, THF, acetonitrile, chloroform, methylene chloride, and mixed solvents thereof, and preferable are THF, DMF or mixtures thereof. The use amount of the solvent is such an amount that the concentration of dissolution of the C-carrier protected peptide is usually 0.3 M to 0.1 mM, preferably 0.2 M to 1 mM.

As the reaction temperature, temperatures generally used in peptide synthesis are used also in the present invention, and for example, the reaction temperature is in a range of usually -20 to 40°C, preferably 0 to 30°C. The reaction time (one cycle) is usually 0.5 to 30 hours.

### 4-2. Step of forming scavenged body of amino acid active ester by amine scavenger

The peptide production method using the present invention is characterized in that after an amino acid coupling step, an amine scavenger is added to the reaction system, and unreacted amino acid active esters are scavenged (captured).

The amine scavenger which can be used in the present invention includes primary or secondary alkylamines, primary or secondary aromatic amines or hydroxylamine. The alkylamine which can be used in the present invention includes, for example, alkylamines having 1 to 14 carbon atoms, and alkylamines having 2 to 10 carbon atoms are preferable, alkylamines having 2 to 8 carbon atoms are more preferable, alkylamines having 3 to 4 carbon atoms are further preferable. The aromatic amine which can be used in the present invention includes, for example, aromatic amines having 1 to 14 carbon atoms, and preferable are aromatic amines having 6 to 10 carbon atoms. Specific amine scavengers include, for example, propylamine, methylamine, hexylamine, aniline, toluidine, 2,4,6-trimethylaniline, anisidine, phenetidine and hydroxylamine, and particularly preferable is propylamine.

The addition amount of the amine scavenger is usually 1 to 30 equivalents, preferably 1 to 15 equivalents, more preferably 2 to 6 equivalents, further preferably 3 to 4 equivalents with respect to the equivalent of the theoretically remaining amino acid. When the addition amount of the amine scavenger is lower than this range, scavenging (capturing) of amino acid active esters becomes insufficient, and an N-Fmoc protected amino acid or peptide cannot be easily removed in the subsequent step, while when the amount is higher than this range, removal of unreacted amine scavengers becomes difficult.

### 4-3. N-terminal de-protection step

In the peptide production method using the method of the present invention, removal of an Fmoc group from an N-Fmoc-C-carrier protected peptide is conducted after scavenging (capturing) an amino acid active ester in the reaction system by an amine scavenger, to form a scavenged body. For removal an Fmoc group from the N-terminal, removal methods generally used in peptide synthesis can be used without restriction also in the present invention. Removal can be conducted using, for example, DBU and piperidine. After this procedure, water washing with an ammonium chloride aqueous solution, a sodium chloride aqueous solution or both of these solutions may be carried out.

### 4-4. Step of crystallization and separation of carrier protected peptide

In the peptide production method using the method of the present invention, a C-carrier protected peptide from which an Fmoc group has been removed can be insolubilized (crystallized) and separated. Insolubilization can be carried out by changing a composition of a solution dissolving a C-carrier protected peptide. Conditions for insolubilization (crystallization) can be appropriately selected depending on the kind of the carrier to be used and the kind and the length of the synthesized C-carrier protected peptide. Examples thereof include means as shown below.

### (Composition changing means)

As the means for changing the composition of a solution which is preferably used in the method of the present invention, means capable of changing a composition of a solution dissolving a C-carrier protected peptide are used without particular restriction. The preferable means for changing a composition of a solution includes, for example, means in which a poor solvent is added to a solution dissolving a C-carrier protected peptide as it is or a poor solvent is added after concentrating the solvent of the solution, to cause crystallization. Here, concentrating means to distill off a part or all of the solvent. Thereafter, the precipitated crystal can be separated, for example, by filtration and centrifugal separation. Impurities separated together with the crystal can be removed completely from the crystallized C-carrier protected peptide, preferably by washing the separated crystal with an organic solvent.

The poor solvent used in the present invention means a solvent in which a C-protected amino acid amide is poorly soluble, that is, a C-protected amino acid amide is not dissolved easily or is not dissolved. For "C-protected amino acid amide is not dissolved easily or is not dissolved", the poor solvent may advantageously be a solvent which is liquid at normal temperature with which the solubility of a C-protected amino acid amide is less than 1% by mass at 25°C, and preferable are acetonitrile, any proportioned aqueous acetonitrile, methanol, any proportioned aqueous methanol and water.

As described above, a process from the condensation reaction (coupling reaction) to the de-protection reaction can be carried out in 1 pot synthesis, if the method of the present invention is used.

### 4-5. Final de-protection step

By removing a carrier at the C-terminal of a C-carrier protected peptide having a desired number of amino acid residues in the final de-protection step, a peptide as the final target can be obtained.

The method of removing a carrier at the C-terminal is not particularly restricted, and de-protection methods known per se may be used.

For example, a de-protection method using TFA can be used, and more specifically, it is preferable to perform de-protection with 50 to 100% trifluoroacetic acid when Ka is used, with 1 to 100% trifluoroacetic acid when Kb is used, with 95 to 100% trifluoroacetic acid when Kc is used, with 1 to 100% trifluoroacetic acid when carrier D and carrier E are used, and with 95 to 100% trifluoroacetic acid when carrier F is used.

The resultant peptide as the final target can be isolated and purified according to methods normally used in peptide chemistry. For example, the reaction mixture can be subjected to extraction washing, crystallization, or chromatography, to isolate and purify a peptide as the final target.

Though the kind of a peptide to be produced by the peptide synthesis method of the present invention is not particularly restricted, it is preferable that the number of amino acid residues of the peptide is, for example, around dozens or lower. The peptide obtained by the peptide synthesis method of the present invention can be used in various fields like existing or unknown synthetic peptides and natural peptides, and the fields include, for example, medicines, foods, cosmetics, and electronic materials.

### [EXAMPLES]

The synthesis method is shown below using peptides having sequences shown below by way of examples, but the present invention is not limited to them.
(peptide A) H-Lys(Boc)Ala-Okb
(peptide B) Fmoc-Gly-Arg(Pbf)-Met-Asp(OtBu)-Arg(Pbf)-Ile-Gly-OH
(peptide C) H-Gln(Trt)-Ser(ψ_{Me,Me}Pro)-Gly-Leu-Gly-Cys(Trt)-Asn(Trt)-Ser(tBu)-Phe-Arg(Pbf)-Tyr(tBu)-OKb
(peptide D) H-D-Arg-Arg-Pro-Hyp-Gly-Thi-Ser-D-Tic-Oic-Arg-OH
(peptide E) H-Ala-Gln(Trt)-Ser(ψ_{Me,Me}Pro)-Gly-Leu-Gly-Cys(Trt)-Asn(Trt)-Ser(tBu)-Phe-Arg(Pbf)-Tyr(tBu)-Leu-OKb
(peptide F) Fmoc-Ser(tBu)-Leu-Arg(Pbf)-Arg(Pbf)-Ser(tBu)-Ser(tBu)-Cys(Trt)-Phe-Gly-OH
(peptide G) HCl•H-Ser(tBu)-Leu-Arg(Pbf)-Arg(Pbf)-Ser(tBu)-Ser(tBu)-Cys(Trt)-Phe-Gly-Gly-Arg(Pbf)-Met-Asp(OtBu)-Arg(Pbf)-Ile-Gly-Ala-Gln(Trt)-Ser(ψ_{Me,Me}Pro)-Gly-Leu-Gly-Cys(Trt)-Asn(Trt)-Ser(tBu)-Phe-Arg(Pbf)-Tyr(tBu)-OKb
(peptide H) H-Ser-Leu-Arg-Arg-Ser-Ser-Ser-Phe-Gly-Gly-Arg-Met-Asp-Arg-Ile-Gly-Gln-Ser-Gly-Leu-Gly-Cys-Asn-Ser-Phe-Arg-Tyr-OH (7Cys-23Cys, SS bond)
(peptide I) H-Glu(OtBu)-Ile-Pro-Glu(OtBu)-Glu(OtBu)-Tyr(tBu)-Leu-OKb
(peptide J) Fmoc-Gly-Gly-Asn(Trt)-Gly-Asp(OtBu)-Phe-Glu(OtBu)-OH
(peptide K) Fmoc-D-Phe-Pro-Arg(Pbf)-Pro-Gly-Gly-OH
(peptide L) H-D-Phe-Pro-Arg(Pbf)-Pro-Gly-Gly-Gly-Gly-Asn(Trt)-Gly-Asp(OtBu)-Phe-Glu(OtBu)-Glu(OtBu)-Ile-Pro-Glu(OtBu)-Glu(OtBu)-Tyr(tBu)-Leu-OKb
(peptide M) H-D-Phe-Pro-Arg-Pro-Gly-Gly-Gly-Gly-Asn-Gly-Asp-Phe-Glu-Glu-Ile-Pro-Glu-Glu-Tyr-Leu-OH
(peptide N) H-Lys(Boc)-Pro-Pro-Ala-Lys(Boc)-Leu-Gln(Trt)-Pro-Arg(Pbf)-OKb
(peptide O) Fmoc-Lys(Boc)Leu-Gln(Trt)-Gln(Trt)-Arg(pbf)-Lys(Boc)-Glu(OtBu)-Ser(tBu)-Lys(Boc)-OH
(peptide P) Boc-Gly-Ser(tBu)-Ser(n-Octanoyl)-Phe-Leu-Ser(tBu)-Pro-Glu(OtBu)-His(Trt)-Gln(Trt)-OH
(peptide Q) Boc-Gly-Ser(tBu)-Ser(n-Octanoyl)-Phe-Leu-Ser(tBu)-Pro-Glu(OtBu)-His(Trt)-Gln(Trt)-Lys(Boc)-Leu-Gln(Trt)-Gln(Trt)-Arg(pbf)-Lys(Boc)-Glu(OtBu)-Ser(tBu)-Lys(Boc)-Lys(Boc)-Pro-Pro-Ala-Lys(Boc)-Leu-Gln(Trt)-Pro-Arg(Pbf)-OKb
(peptide R) H-Gly-Ser-Ser(n-Octanoyl)-Phe-Leu-Ser-Pro-Glu-His-Gln-Lys-Leu-Gln-Gln-Arg-Lys-Glu-Ser-Lys-Lys-Pro-Pro-Ala-Lys-Leu-Gln-Pro-Arg-OH

In the present specification and examples described below, the following abbreviations are used.
AAs: one or more any amino acid residues
AAx: any amino acid residue
Boc: tert-butoxycarbonyl
COMU (1-cyano-2-ethoxy-2-oxoethylideneaminooxy)dimethylamino-morpholino-carbeniumhexafluoro phosphate
CPME: cyclopentyl methyl ether
DBU: 1,8-diazabicyclo[5.4.0]-7-undecene
DCM: dichloromethane
DIPCI: diisopropylcarbodiimide
DMAP: N,N-dimethyl-4-aminopyridine
DMF: N,N-dimethylformamide
DMT-MM: 4-(4,6-dimethoxy-1,3,5-triazin-2-yl)-4-methylmorphonium chloride EDT: 1,2-ethanedithiol
HATU: O-(7-azabenzotriazol-1-yl)-1,1,3,3-tetramethyluroniumhexafluoro phosphate
HBTU: O-(benzotriazol-1-yl)-1,1,3,3-tetramethyluroniumhexafluoro phosphate
HOAt: 1-hydroxy-7-azabenzotriazole
HOBt: 1-hydroxybenzotriazole
Ka: 3,4,5-trioctadecyloxybenzyl
Kb: 2,4-didocosyloxybenzyl
Kc: 3,5-didocosyloxybenzyl
Me: methyl
Oxyma: cyano(hydroxyimino)ethyl acetate
Pbf: 2,2,4,6,7-pentamethyldihydrobenzofuran-5-sulfonyl
Su: succinimidyl
tBu: tert-butyl
TFA: trifluoroacetic acid
TFE: 2,2,2-trifluoroethanol
THF: tetrahydrofuran
TIS: triisopropylsilane
WSC•HCl: 1-ethyl-3-(3-dimethylaminopropyl)carbodiimide hydrochloride

### (General synthesis method)

General synthesis methods used in the present examples are shown below.

### (1) De-Fmoc (Fmoc-deletion) general synthesis method

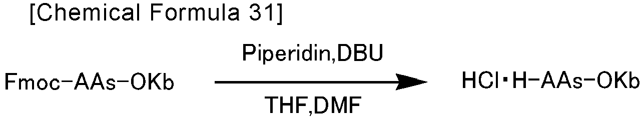

A starting raw material was dissolved in a mixture of THF:DMF (9/1) so as to give a concentration of 0.05 M, and piperidine (1.5 equiv) and DBU (1 equiv) were added and the mixture was stirred at room temperature for 10 minutes. Concentrated hydrochloric acid was added until pH of the reaction mixture became around 6 and the solvent was distilled off under reduced pressure. To the residue was added a mixture of acetonitrile:water = 9:1 and the deposited precipitate was filtrated, and suspended and washed with a mixed liquid of acetonitrile:water = 9:1, further, suspension washing with acetonitrile was conducted, and the resultant solid was dried under reduced pressure, to obtain a de-Fmoc body.

### (2) Amino acid condensation general synthesis method

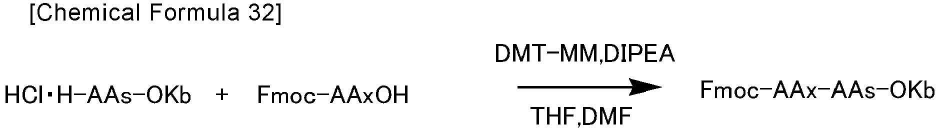

Starting raw materials were dissolved in a mixture of THF:DMF (9/1) so as to give a concentration of 0.05 M, and Fmoc-AAx-OH (1.3 equiv), DMT-MM (1.13 equiv) and DIPEA (1.2 equiv) were added and the mixture was stirred at room temperature for 30 minutes. The solvent was distilled off under reduced pressure, to the residue was added acetonitrile and the deposited precipitate was filtrated, suspension washing with acetonitrile was conducted twice, and the resultant solid was dried under reduced pressure, to obtain an amino acid condensate.

De-protection methods in the presence of an amine scavenger characteristic in the method of the present invention, used in the examples of the present invention, are shown below. Examples using propylamine as an example of the amine scavenger are shown.

### (3) 1 pot condensation de-protection method with addition of amine scavenger

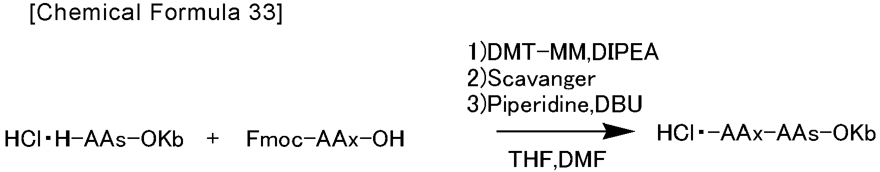

Starting raw materials were dissolved in a mixture of THF:DMF (9/1) so as to give a concentration of 0.05 M, and Fmoc-AAx-OH (1.3 equiv), DMT-MM (1.13 equiv) and DIPEA (1.13 equiv) were added and the mixture was stirred at room temperature for 30 minutes. As the amine scavenger, propylamine (1.2 equiv) was added and the mixture was stirred at room temperature for 30 minutes. HOBT (1 equiv), piperidine (1.5 equiv) and DBU (8 equiv) were added and the mixture was stirred at room temperature for 10 minutes. Concentrated hydrochloric acid was added until pH of the reaction mixture became around 6 and the solvent was distilled off under reduced pressure. To the residue was added a mixed liquid of acetonitrile:water=1:1 and the deposited precipitate was filtrated, and suspended and washed with a mixture of acetonitrile:water = 9:1, further, suspension washing with acetonitrile was conducted, and the resultant solid was dried under reduced pressure, to obtain an amino acid condensate.

### (4) 1 pot condensation de-protection method in which amine scavenger is added and washing with ammonium chloride aqueous solution is conducted

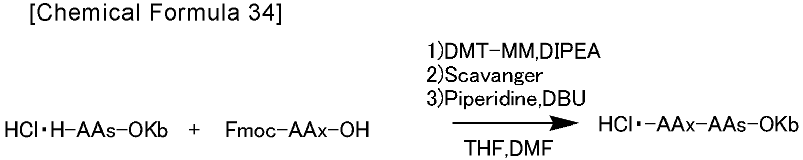

Starting raw materials were dissolved in a mixed liquid of THF:DMF (9/1) so as to give a concentration of 0.05 M, and Fmoc-AAx-OH (1.3 equiv), DMT-MM (1.13 equiv) and DIPEA (1.13 equiv) were added and the mixture was stirred at room temperature for 30 minutes. As the amine scavenger, propylamine (1.2 equiv) was added and the mixture was stirred at room temperature for 30 minutes. HOBT (1 equiv), piperidine (1.5 equiv) and DBU (8 equiv) were added and the mixture was stirred at room temperature for 10 minutes. Concentrated hydrochloric acid was added until pH of the reaction mixture became around 6. A solution of saturated ammonium chloride aqueous solution:water = 1:2 of double amount of the reaction solvent was added to the reaction mixture, and the liquid was washed and separated, and the aqueous layer was discarded. Further, a solution of saturated saline:water = 1:2 of double amount of the reaction solvent was added, and the liquid was washed and separated, and the aqueous layer was discarded. Further, saturated saline of double amount of the reaction solvent was added, and the liquid was washed and separated, and the aqueous layer was discarded. The resultant organic layer was held under reduced pressure and the solvent was distilled off. To the residue was added a mixture of acetonitrile:water = 1:1 and the deposited precipitate was filtrated, and suspended and washed with a mixture of acetonitrile:water = 9:1, further, suspension washing with acetonitrile was conducted, and the resultant solid was dried under reduced pressure, to obtain an amino acid condensate.

Next, a method of de-protecting (cutting) a carrier used in the present invention from a peptide is shown.

### (5) Kb protective group general de-protection method

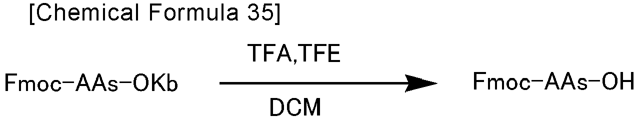

A raw material was dissolved in DCM so as to give a concentration of 0.01 M, and TFE of 1/10 amount of the DCM was added, further, TFA of 1/100 amount of the DCM was added and the mixture was stirred at room temperature for 30 minutes. The precipitate was filtrated, and the filtrate was adjusted to pH=9 with DIPEA, and the solvent was distilled off under reduced pressure. To the residue was added water and the deposited precipitate was filtrated, the resultant solid was dissolved in THF and toluene, then, distillation off under reduced pressure was performed. Suspension washing with addition of diisopropyl ether to the resultant solid was repeated three times, and the resultant solid was dried under reduced pressure, to obtain a de-Kb protected body.

Peptide synthesis using the method of the present invention is shown below.

### (Example 1) Synthesis of intermediate (compound 2)

### Synthesis of compound 1

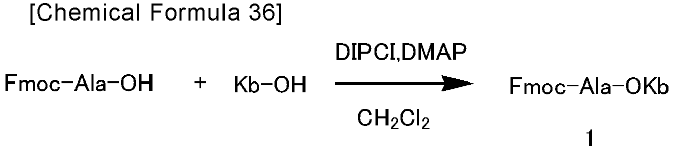

2,4-didocosyloxybenzyl alcohol (expressed as "Kb-OH")(3.79 g, 5.00 mmol) was dissolved in DCM (50 ml), and Fmoc-Ala-OH (2.34 g, 7.50 mmol, 1.5 equiv), DIPCI (1169 µL, 7.50 mmol, 1.5 equiv) and DMAP (31 mg, 0.25 mmol, 0.05 equiv) were added and the mixture was stirred at room temperature for 30 minutes. Propylamine (411 µL, 5.00 mmol, 1.0 equiv) was added and the mixture was stirred at room temperature for 30 minutes. The precipitate was filtrated, and the filtrate was distilled off under reduced pressure. To the residue was added MeOH and the deposited precipitate was filtrated, suspension washing with MeOH was conducted twice, suspension washing with acetonitrile was conducted, and the resultant solid was dried under reduced pressure, to obtain a compound 1 (5.02 g, 95.5%).

### Synthesis of compound 2

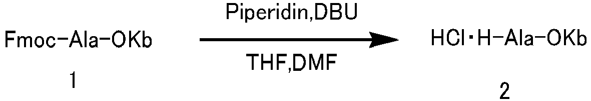

The compound 1 (5.00 g, 4.76 mmol) was dissolved in a mixture of THF (86 ml) and DMF (10 ml), and piperidine (707 µL, 7.14 mmol, 1.5 equiv) and DBU (712 µL, 4.76 mmol, 1 equiv) were added and the mixture was stirred at room temperature for 10 minutes. Concentrated hydrochloric acid was added until pH of the reaction mixture became around 6 and the solvent was distilled off under reduced pressure. To the residue was added a mixture of acetonitrile:water = 9:1 and the deposited precipitate was filtrated, and suspended and washed with a mixture of acetonitrile:water = 9:1, further, suspension washing with acetonitrile was conducted, and the resultant solid was dried under reduced pressure, to obtain a compound 2 (4.05 g, 98.3%).

### (Comparative Example 1) Two-step synthesis of H-Lys(Boc)Ala-OKb (compound 4) Synthesis of compound 3

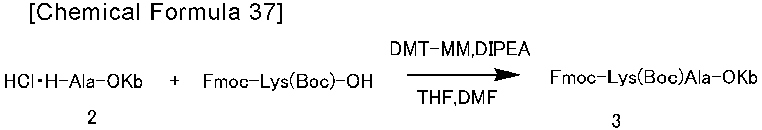

The compound 2 (86 mg, 0.1 mmol) obtained in Example 1 was dissolved in a mixture of THF (2 ml) and DMF (0.2 ml), and Fmoc-Lys(Boc)-OH (61 mg, 0.13 mmol, 1.3 equiv), DMT-MM (32 mg, 0.113 mmol, 1.13 equiv) and DIPEA (21 µL, 0.12 mmol, 1.2 equiv) were added and the mixture was stirred at room temperature for 30 minutes. The solvent was distilled off under reduced pressure, to the residue was added acetonitrile and the deposited precipitate was filtrated, suspension washing with acetonitrile was conducted twice, and the resultant solid was dried under reduced pressure, to obtain a compound 3 (128.5 mg, quant).

### Synthesis of compound 4

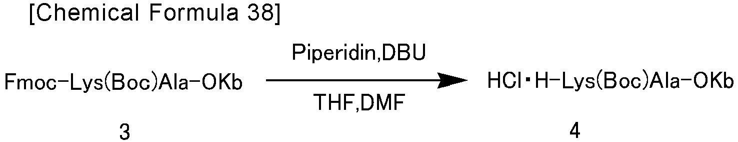

The compound 3 (119 mg, 0.093 mmol) was dissolved in a mixture of THF (2 ml) and DMF (0.2 ml), and piperidine (14 µL, 0.14 mmol, 1.5 equiv) and DBU (28 µL, 0.28 mmol, 2 equiv) were added and the mixture was stirred at room temperature for 10 minutes. Concentrated hydrochloric acid was added until pH of the reaction mixture became around 6 and the solvent was distilled off under reduced pressure. To the residue was added a mixture of acetonitrile:water = 1:1 and the deposited precipitate was filtrated, and suspended and washed with a mixture of acetonitrile:water = 9:1, further, suspension washing with acetonitrile was conducted, and the resultant solid was dried under reduced pressure, to obtain a compound 4 (92.5 mg, 90.7%).

### (Example 2) Synthesis of H-Lys(Boc)Ala-OKb (compound 4) with addition of amine scavenger -(1)

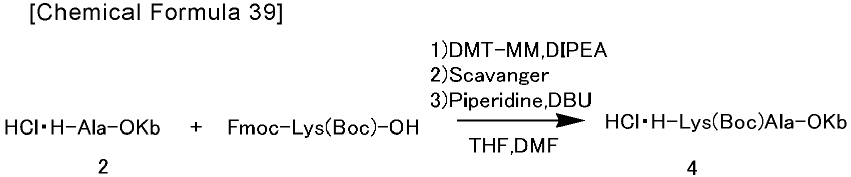

The compound 2 (173 mg, 0.2 mmol) obtained in Example 1 was dissolved in a mixture of THF (4 ml) and DMF (0.4 ml), and Fmoc-Lys(Boc)-OH (122 mg, 0.26 mmol, 1.3 equiv), DMT-MM (65 mg, 0.226 mmol, 1.13 equiv) and DIPEA (42 µL, 0.24 mmol, 1.2 equiv) were added and the mixture was stirred at room temperature for 30 minutes. As the amine scavenger, propylamine (19.7 µL, 0.24 mmol, 1.2 equiv) was added and the mixture was stirred at room temperature for 30 minutes. HOBT (30.6 mg, 0.26 mmol, 1 equiv), piperidine (30 µL, 0.3 mmol, 1.5 equiv) and DBU (239 µL, 1.6 mmol, 8 equiv) were added and the mixture was stirred at room temperature for 10 minutes. Concentrated hydrochloric acid was added until pH of the reaction mixture became around 6 and the solvent was distilled off under reduced pressure. To the residue was added a mixture of acetonitrile:water = 1:1 and the deposited precipitate was filtrated, and suspended and washed with a mixture of acetonitrile:water = 9:1, further, suspension washing with acetonitrile was conducted, and the resultant solid was dried under reduced pressure, to obtain a compound 4 (92.5 mg, 90.7%).

### (Example 3) Synthesis of H-Lys(Boc)Lys(Boc)Ala-OKb (compound 5) with addition of amine scavenger

### [Chemical Formula 40]

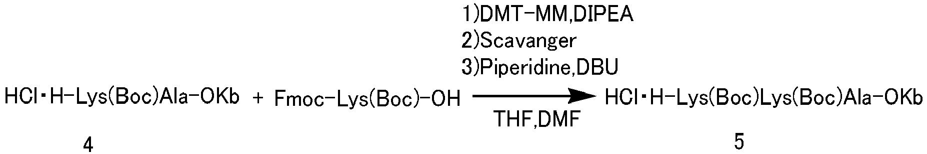

The compound 4 (109 mg, 0.1 mmol) obtained in Example 2 was dissolved in a mixture of THF (2 ml) and DMF (0.2 ml), and Fmoc-Lys(Boc)-OH (61 mg, 0.13 mmol, 1.3 equiv), DMT-MM (32 mg, 0.113 mmol, 1.13 equiv) and DIPEA (21 µL, 0.12 mmol, 1.2 equiv) were added and the mixture was stirred at room temperature for 30 minutes. As the amine scavenger, propylamine (9.9 µL, 0.12 mmol, 1.2 equiv) was added and the mixture was stirred at room temperature for 30 minutes. Piperidine (15 µL, 0.15 mmol, 1.5 equiv) and DBU (105 µL, 1.4 mmol, 7 equiv) were added and the mixture was stirred at room temperature for 10 minutes. After this, the same procedure as in Example 2 was conducted, to obtain a compound 5 (115.2 mg, 87.2%).

### (Example 4) Synthesis of H-Lys(Boc)Ala-OKb (compound 4) with addition of amine scavenger -(2)

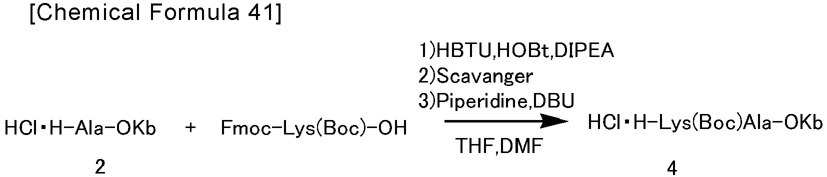

The compound 2 (86 mg, 0.1 mmol) obtained in Example 1 was dissolved in a mixture of THF (2 ml) and DMF (0.2 ml), and Fmoc-Lys(Boc)-OH (61 mg, 0.13 mmol, 1.3 equiv), HBTU (44 mg, 0.113 mmol, 1.13 equiv), HOBt (18 mg, 0.113 mmol, 1.13 equiv) and DIPEA (87 µL, 0.5 mmol, 5 equiv) were added and the mixture was stirred at room temperature for 30 minutes. As the amine scavenger, propylamine (9.9 µL, 0.12 mmol, 1.2 equiv) was added and the mixture was stirred at room temperature for 30 minutes. Piperidine (15 µL, 0.15 mmol, 1.5 equiv) and DBU (120 µL, 1.6 mmol, 8 equiv) were added and the mixture was stirred at room temperature for 10 minutes. After this, the same procedure as in Example 2 was conducted, to obtain a compound 4 (109.2 mg, 99.9%).

### (Example 5) Synthesis of H-Lys(Boc)Ala-OKb (compound 4) with addition of amine scavenger -(3)

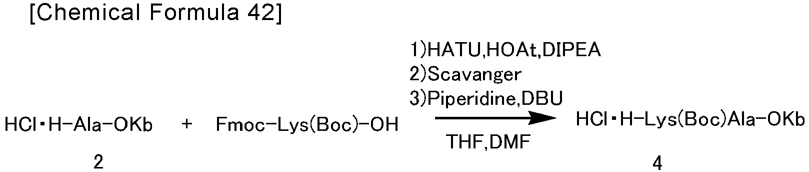

The compound 2 (86 mg, 0.1 mmol) obtained in Example 1 was dissolved in a mixture of THF (2 ml) and DMF (0.2 ml), and Fmoc-Lys(Boc)-OH (61 mg, 0.13 mmol, 1.3 equiv), HATU (44 mg, 0.113 mmol, 1.13 equiv), HOAt (16 mg, 0.113 mmol, 1.13 equiv) and DIPEA (87 µL, 0.5 mmol, 5 equiv) were added and the mixture was stirred at room temperature for 30 minutes. As the amine scavenger, propylamine (9.9 µL, 0.12 mmol, 1.2 equiv) was added and the mixture was stirred at room temperature for 30 minutes. Piperidine (15 µL, 0.15 mmol, 1.5 equiv) and DBU (120 µL, 1.6 mmol, 8 equiv) were added and the mixture was stirred at room temperature for 10 minutes. After this, the same procedure as in Example 2 was conducted, to obtain a compound 4 (103.5 mg, 94.7%).

### (Example 6) Synthesis of H-Lys(Boc)Ala-OKb (compound 4) with addition of amine scavenger -(4)

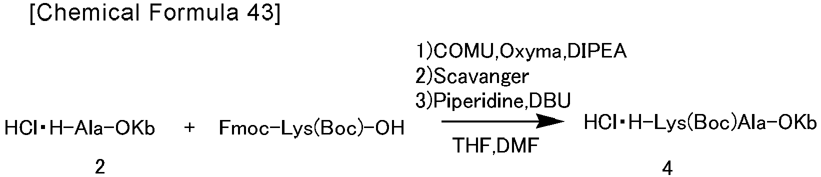

The compound 2 (86 mg, 0.1 mmol) obtained in Example 1 was dissolved in a mixture of THF (2 ml) and DMF (0.2 ml), Fmoc-Lys(Boc)-OH (61 mg, 0.13 mmol, 1.3 equiv), COMU (50 mg, 0.113 mmol, 1.13 equiv), Oxyma (17 mg, 0.113 mmol, 1.13 equiv) and DIPEA (87 µL, 0.5 mmol, 5 equiv) were added and the mixture was stirred at room temperature for 30 minutes. As the amine scavenger, propylamine (9.9 µL, 0.12 mmol, 1.2 equiv) was added and the mixture was stirred at room temperature for 30 minutes. Piperidine (15 µL, 0.15 mmol, 1.5 equiv) and DBU (120 µL, 1.6 mmol, 8 equiv) were added and the mixture was stirred at room temperature for 10 minutes. After this, the same procedure as in Example 2 was conducted, to obtain a compound 4 (105.7 mg, 96.7%).

### (Example 7) Synthesis of H-Lys(Boc)Ala-OKb (compound 4) with addition of amine scavenger -(5)

The compound 2 (86 mg, 0.1 mmol) obtained in Example 1 was dissolved in a mixture of THF (2 ml) and DMF (0.1 ml), Fmoc-Lys(Boc)-OH (61 mg, 0.13 mmol, 1.3 equiv), DMT-MM (32 mg, 0.113 mmol, 1.13 equiv) and DIPEA (21 µL, 0.12 mmol, 1.2 equiv) were added and the mixture was stirred at room temperature for 30 minutes. As the amine scavenger, propylamine (2.5 µL, 0.03 mmol, 0.3 equiv) was added and the mixture was stirred at room temperature for 30 minutes. HOBT (30.6 mg, 0.26 mmol, 1 equiv), piperidine (30 µL, 0.3 mmol, 1.5 equiv) and DBU (239 µL, 1.6 mmol, 8 equiv) were added and the mixture was stirred at room temperature for 10 minutes. After this, the same procedure as in Example 2 was conducted, to obtain a compound 4 (105.3 mg, 96.3%).

### (Example 8) Synthesis of H-Lys(Boc)Ala-OKb (compound 4) with addition of amine scavenger -(6)

The compound 2 (86 mg, 0.1 mmol) obtained in Example 1 was dissolved in a mixture of THF (2 ml) and DMF (0.1 ml), Fmoc-Lys(Boc)-OH (61 mg, 0.13 mmol, 1.3 equiv), DMT-MM (32 mg, 0.113 mmol, 1.13 equiv) and DIPEA (21 µL, 0.12 mmol, 1.2 equiv) were added and the mixture was stirred at room temperature for 30 minutes. As the amine scavenger, propylamine (4.9 µL, 0.06 mmol, 0.6 equiv) was added and the mixture was stirred at room temperature for 30 minutes. HOBT (30.6 mg, 0.26 mmol, 1 equiv), piperidine (30 µL, 0.3 mmol, 1.5 equiv) and DBU (239 µL, 1.6 mmol, 8 equiv) were added and the mixture was stirred at room temperature for 10 minutes. After this, the same procedure as in Example 2 was conducted, to obtain a compound 4 (105.6 mg, 96.6%).

### (Example 9) Synthesis of H-Lys(Boc)Ala-OKb (compound 4) with addition of amine scavenger -(7)

The compound 2 (86 mg, 0.1 mmol) obtained in Example 1 was dissolved in a mixture of THF (2 ml) and DMF (0.1 ml), Fmoc-Lys(Boc)-OH (61 mg, 0.13 mmol, 1.3 equiv), DMT-MM (32 mg, 0.113 mmol, 1.13 equiv) and DIPEA (21 µL, 0.12 mmol, 1.2 equiv) were added and the mixture was stirred at room temperature for 30 minutes. As the amine scavenger, propylamine (19.7 µL, 0.24 mmol, 2.4 equiv) was added and the mixture was stirred at room temperature for 30 minutes. HOBT (30.6 mg, 0.26 mmol, 1 equiv), piperidine (30 µL, 0.3 mmol, 1.5 equiv) and DBU (239 µL, 1.6 mmol, 8 equiv) were added and the mixture was stirred at room temperature for 10 minutes. After this, the same procedure as in Example 2 was conducted, to obtain a compound 4 (105.6 mg, 96.6%).

### (Example 10) Synthesis of H-Lys(Boc)Ala-OKb (compound 4) with addition of amine scavenger -(8)

The compound 2 (86 mg, 0.1 mmol) obtained in Example 1 was dissolved in a mixture of THF (2 ml) and DMF (0.1 ml), Fmoc-Lys(Boc)-OH (61 mg, 0.13 mmol, 1.3 equiv), DMT-MM (32 mg, 0.113 mmol, 1.13 equiv) and DIPEA (21 µL, 0.12 mmol, 1.2 equiv) were added and the mixture was stirred at room temperature for 30 minutes. As the amine scavenger, propylamine (37 µL, 0.45 mmol, 4.5 equiv) was added and the mixture was stirred at room temperature for 30 minutes. HOBT (30.6 mg, 0.26 mmol, 1 equiv), piperidine (30 µL, 0.3 mmol, 1.5 equiv) and DBU (239 µL, 1.6 mmol, 8 equiv) were added and the mixture was stirred at room temperature for 10 minutes. After this, the same procedure as in Example 2 was conducted, to obtain a compound 4 (105.2 mg, 96.2%).

### (Example 11) Synthesis of H-Lys(Boc)Ala-OKb (compound 4) with addition of amine scavenger -(9)

The compound 2 (86 mg, 0.1 mmol) obtained in Example 1 was dissolved in a mixture of THF (2 ml) and DMF (0.1 ml), Fmoc-Lys(Boc)-OH (61 mg, 0.13 mmol, 1.3 equiv), DMT-MM (32 mg, 0.113 mmol, 1.13 equiv) and DIPEA (21 µL, 0.12 mmol, 1.2 equiv) were added and the mixture was stirred at room temperature for 30 minutes. As the amine scavenger, a 40% methylamine aqueous solution (10.3 µL, 0.12 mmol, 1.2 equiv) was added and the mixture was stirred at room temperature for 30 minutes. HOBT (30.6 mg, 0.26 mmol, 1 equiv), piperidine (30 µL, 0.3 mmol, 1.5 equiv) and DBU (239 µL, 1.6 mmol, 8 equiv) were added and the mixture was stirred at room temperature for 10 minutes. After this, the same procedure as in Example 2 was conducted, to obtain a compound 4 (101.6 mg, 93.0%).

### (Example 12) Synthesis of H-Lys(Boc)Ala-OKb (compound 4) with addition of amine scavenger -(10)

The compound 2 (86 mg, 0.1 mmol) obtained in Example 1 was dissolved in a mixture of THF (2 ml) and DMF (0.1 ml), Fmoc-Lys(Boc)-OH (61 mg, 0.13 mmol, 1.3 equiv), DMT-MM (32 mg, 0.113 mmol, 1.13 equiv) and DIPEA (21 µL, 0.12 mmol, 1.2 equiv) were added and the mixture was stirred at room temperature for 30 minutes. As the amine scavenger, a 50% hydroxylamine aqueous solution (7.1 µL, 0.12 mmol, 1.2 equiv) was added and the mixture was stirred at room temperature for 30 minutes. HOBT (30.6 mg, 0.26 mmol, 1 equiv), piperidine (30 µL, 0.3 mmol, 1.5 equiv) and DBU (239 µL, 1.6 mmol, 8 equiv) were added and the mixture was stirred at room temperature for 10 minutes. After this, the same procedure as in Example 2 was conducted, to obtain a compound 4 (104.1 mg, 95.2%).

### (Example 13) Synthesis of H-Lys(Boc)Ala-OKb (compound 4) with addition of amine scavenger -(11)

The compound 2 (86 mg, 0.1 mmol) obtained in Example 1 was dissolved in a mixture of THF (2 ml) and DMF (0.1 ml), Fmoc-Lys(Boc)-OH (61 mg, 0.13 mmol, 1.3 equiv), DMT-MM (32 mg, 0.113 mmol, 1.13 equiv) and DIPEA (21 µL, 0.12 mmol, 1.2 equiv) were added and the mixture was stirred at room temperature for 30 minutes. As the amine scavenger, hexylamine (15.9 µL, 0.12 mmol, 1.2 equiv) was added and the mixture was stirred at room temperature for 30 minutes. HOBT (30.6 mg, 0.26 mmol, 1 equiv), piperidine (30 µL, 0.3 mmol, 1.5 equiv) and DBU (239 µL, 1.6 mmol, 8 equiv) were added and the mixture was stirred at room temperature for 10 minutes. After this, the same procedure as in Example 2 was conducted, to obtain a compound 4 (104.6 mg, 95.7%).

### (Example 14) Synthesis of H-Lys(Boc)Ala-OKb (compound 4) with addition of amine scavenger -(12)

The compound 2 (86 mg, 0.1 mmol) obtained in Example 1 was dissolved in a mixture of THF (2 ml) and DMF (0.1 ml), Fmoc-Lys(Boc)-OH (61 mg, 0.13 mmol, 1.3 equiv), DMT-MM (32 mg, 0.113 mmol, 1.13 equiv) and DIPEA (21 µL, 0.12 mmol, 1.2 equiv) were added and the mixture was stirred at room temperature for 30 minutes. As the amine scavenger, decylamine (15.9 µL, 0.12 mmol, 1.2 equiv) was added and the mixture was stirred at room temperature for 30 minutes. HOBT (30.6 mg, 0.26 mmol, 1 equiv), piperidine (30 µL, 0.3 mmol, 1.5 equiv) and DBU (239 µL, 1.6 mmol, 8 equiv) were added and the mixture was stirred at room temperature for 10 minutes. After this, the same procedure as in Example 2 was conducted, to obtain a compound 4 (107.5 mg, 98.4%).

### (Comparative Example 2) Synthesis of H-Lys(Boc)Ala-OKb (compound 4) with addition of amine scavenger not included in the present invention -(13)

The compound 2 (86 mg, 0.1 mmol) obtained in Example 1 was dissolved in a mixture of THF (2 ml) and DMF (0.1 ml), Fmoc-Lys(Boc)-OH (61 mg, 0.13 mmol, 1.3 equiv), DMT-MM (32 mg, 0.113 mmol, 1.13 equiv) and DIPEA (21 µL, 0.12 mmol, 1.2 equiv) were added and the mixture was stirred at room temperature for 30 minutes. As the amine scavenger, 2-aminoethanol (7.2 µL, 0.12 mmol, 1.2 equiv) was added and the mixture was stirred at room temperature for 30 minutes. HOBT (30.6 mg, 0.26 mmol, 1 equiv), piperidine (30 µL, 0.3 mmol, 1.5 equiv) and DBU (239 µL, 1.6 mmol, 8 equiv) were added and the mixture was stirred at room temperature for 10 minutes. After this, the same procedure as in Example 2 was conducted, to obtain a compound 4 (103.6 mg, 94.8%).

### (Example 15) Synthesis of H-Lys(Boc)Ala-OKb (compound 4) with addition of amine scavenger -(14)

The compound 2 (86 mg, 0.1 mmol) obtained in Example 1 was dissolved in a mixture of THF (2 ml) and DMF (0.1 ml), Fmoc-Lys(Boc)-OH (187 mg, 0.4 mmol, 4.0 equiv), DMT-MM (100 mg, 0.36 mmol, 3.6 equiv) and DIPEA (21 µL, 0.12 mmol, 1.2 equiv) were added and the mixture was stirred at room temperature for 30 minutes. As the amine scavenger, propylamine (99 µL, 1.2 mmol, 12 equiv) was added and the mixture was stirred at room temperature for 30 minutes. HOBT (30.6 mg, 0.26 mmol, 1 equiv), piperidine (30 µL, 0.3 mmol, 1.5 equiv) and DBU (239 µL, 1.6 mmol, 8 equiv) were added and the mixture was stirred at room temperature for 10 minutes. After this, the same procedure as in Example 2 was conducted, to obtain a compound 4 (104.2 mg, 95.3%).

### (Example 16) Measurement of purity of H-Ala-OKb (compound 2), H-Lys(Boc)Ala-OKb (compound 4) and H-Lys(Boc)Lys(Boc)Ala-OKb (compound 5) by HPLC

### (1) Sample preparation (common)

Each 1 mg of the compounds obtained in Examples and Comparative Examples described above were weighed and dissolved in 0.5 mL of THF, and a 0.1M Fmoc-OSu THF solution (20 µL, 0.02 mmol, about 2 equiv) and a 0.2M DIPEA THF solution (10 µL, 0.02 mmol, about 2 equiv) were added and the mixtures were stirred at room temperature for 1 hour. After air drying, the solutions were suspended and washed with acetonitrile three times, and dried under reduced pressure. To the resultant solids was added 1 ml of THF for HPLC (containing no stabilizer) and the resultant solutions were filtrated through a 0.2 µm membrane filter, and the resultant filtrates were used as samples for HPLC and subjected to measurement of HPLC.

### (2) HPLC measurement

HPLC measurement was conducted according to the following conditions.

### <Reverse phase HPLC>

Column: reverse phase C4 column
Mobile phase A: THF:CH₃CN = 8:2 mixture
Mobile phase B: 0.1% TFA-containing water
Flow rate: 0.2 ml/min
Gradient: A, from 70% to 100% over 30 minutes
Detection wavelength: 254 nm

### <Normal phase HPLC>

Column: normal phase silica gel column
Mobile phase A: ethyl acetate
Mobile phase B: n-hexane
Flow rate: 0.2 ml/min
Gradient: mobile phase A, from 0% to 100% over 30 minutes
Detection wavelength: 280 nm

The results are shown in Table 1 below.

**[Table 1]**

| | **Compound 2 contained amount ( reverse phase )** | **Compound 4 contained amount ( reverse phase )** | **Compound 5 contained amount ( normal phase )** |
|---|---|---|---|
| **Example 1** | **99.35%** | **0** | **0** |
| **Comparative Example 1** | **0** | **99.46%** | **0.05%** |
| **Example 2** | **0** | **99.12%** | **0** |
| **Example 3** | **0** | **0** | **100%** |
| **Example 4** | **0** | **98.36%** | **0** |
| **Example 5** | **0** | **99.03%** | **0** |
| **Example 6** | **0** | **99.29%** | **0** |
| **Example 7** | **0** | **99.78%** | **0** |
| **Example 8** | **0** | **99.71%** | **0** |
| **Example 9** | **0** | **99.78%** | **0** |
| **Example 10** | **0** | **99.74%** | **0** |
| **Example 11** | **0** | **99.29%** | **0.03%** |
| **Example 12** | **0** | **99.31%** | **0** |
| **Example 13** | **0** | **98.26%** | **0** |
| **Example 14** | **0** | **99.21%** | **0** |
| **Example 15** | **0** | **98.42%** | **0** |
| **Comparative Example 2** | **0** | **99.54%** | **0.20%** |

### (Example 17) Synthesis of HCl•H-Ile-Gly-OKb (compound 14)

### Synthesis of compound 6

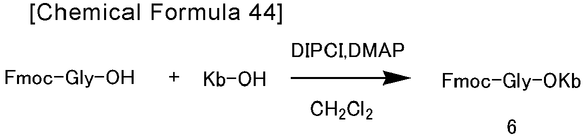

2,4-didocosyloxybenzyl alcohol (expressed as "KbOH")(1.51 g, 2.00 mmol) was dissolved in DCM (50 ml), Fmoc-Gly-OH (892 mg, 3.0 mmol, 1.5 equiv), DIPCI (467 µL, 3.0 mmol, 1.5 equiv) and DMAP (12 mg, 0.1 mmol, 0.05 equiv) were added and the mixture was stirred at room temperature for 30 minutes. The precipitate was filtrated, and the filtrate was distilled off under reduced pressure. To the residue was added MeOH and the deposited precipitate was filtrated, suspension washing with MeOH was conducted twice, suspension washing with acetonitrile was conducted, and the resultant solid was dried under reduced pressure, to obtain a compound 6 (2.01 g, 97.1%).

### Synthesis of compound 7

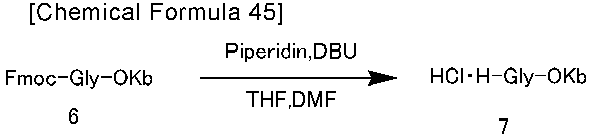

The resultant compound 6 (2.01 g, 0.1 mmol) was subjected to a de-Fmoc operation according to a de-Fmoc general synthesis method, to obtain a compound 7 (1.84 g, quant).

### Synthesis of compound 8

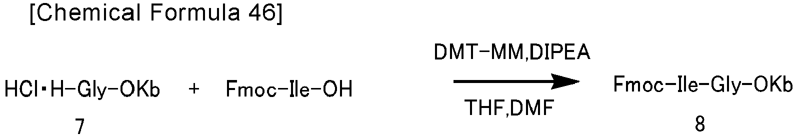

The resultant compound 7 (1.84 g, 0.1 mmol) was condensed with Fmoc-Ile-OH according to an amino acid general condensation method, to obtain a compound 8 (1.99 g, 88.9%).

### Synthesis of compound 9

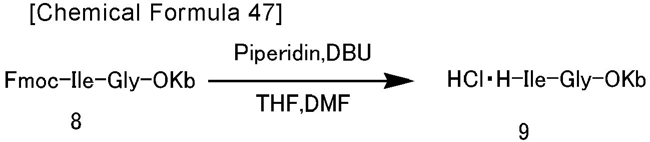

The resultant compound 8 (662 mg, 0.58 mmol) was subjected to a de-Fmoc operation according to a de-Fmoc general synthesis method, to obtain a compound 9 (505 mg, 91%).

### (Comparative Example 3) Synthesis of Fmoc-Gly-Arg(Pbf)-Met-Asp(OtBu)-Arg(Pbf)-Ile-Gly-OKb (compound 10)

The following amino acids were introduced into the compound 9 obtained in Example 17 by repeating an amino acid general condensation method and a de-Fmoc general synthesis method, to obtain a compound 10.
Third residue: Fmoc-Arg(Pbf)-OH
Fourth residue: Fmoc-Asp(OtBu)-OH
Fifth residue: Fmoc-Met-OH
Sixth residue: Fmoc-Arg(Pbf)-OH
Seventh residue: Fmoc-Gly-OH

### (Comparative Example 4) Synthesis of Fmoc-Gly-Arg(Pbf)-Met-Asp(OtBu)-Arg(Pbf)-Ile-Gly-OH (compound 11)

The compound 10 obtained in Comparative Example 2 was subjected to a de-Kb operation according to a Kb protective group general de-protection method, to obtain a compound 11. The resultant compound was subjected to LC-MS measurement, to observe 1643.7 [M+Gly+H⁺], 1995.2 [M+Arg(Pbf)+H⁺], 1758.1 [M+Asp(OtBu)+H⁺] and 1718.0 [M+Met+H⁺].

### (Example 18) Synthesis of Fmoc-Gly-Arg(Pbf)-Met-Asp(OtBu)-Arg(Pbf)-Ile-Gly-OKb (compound 10) by 1 pot condensation de-protection method with addition of amine scavenger

The following amino acids were introduced into the compound 9 obtained in Example 17 by repeating a 1 pot condensation de-protection method with addition of an amine scavenger, to obtain a compound 10.
Introduction of
Third residue: Fmoc-Arg(Pbf)-OH
Fourth residue: Fmoc-Asp(OtBu)-OH
Fifth residue: Fmoc-Met-OH
Sixth residue: Fmoc-Arg(Pbf)-OH
Seventh residue: Fmoc-Gly-OH, which was conducted according to an amino acid general condensation method.

### (Example 19) Synthesis of Fmoc-Gly-Arg(Pbf)-Met-Asp(OtBu)-Arg(Pbf)-Ile-Gly-OH (compound 11)

The compound 10 obtained in Example 18 was subjected to a de-Kb operation according to a Kb protective group general de-protection method, to obtain a compound 11. The resultant compound was subjected to LC-MS measurement, but 1643.7 [M+Gly+H⁺], 1995.2 [M+Arg(Pbf)+H⁺], 1758.1 [M+Asp(OtBu)+H⁺] and 1718.0 [M+Met+H⁺] were not observed.

### (Example 20) Synthesis of H-Gly-Leu-Gly-Cys(Trt)-Asn(Trt)-Ser(tBu)-Phe-Arg(Pbf)-Tyr(tBu)-Okb (compound 14)

### Synthesis of compound 12

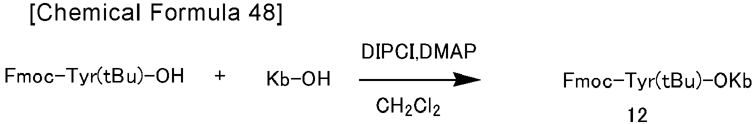

2,4-didocosyloxybenzyl alcohol (expressed as "Kb-OH")(3.79 g, 5.00 mmol) was dissolved in DCM (50 ml), Fmoc-Tyr(tBu)-OH (3.45 g, 7.50 mmol, 1.5 equiv), DIPCI (1169 µL, 7.50 mmol, 1.5 equiv) and DMAP (31 mg, 0.25 mmol, 0.05 equiv) were added and the mixture was stirred at room temperature for 30 minutes. Propylamine (811 µL, 10.0 mmol, 2.0 equiv) was added and the mixture was stirred at room temperature for 30 minutes. The precipitate was filtrated, and the filtrate was distilled off under reduced pressure. To the residue was added MeOH and the deposited precipitate was filtrated, suspension washing with MeOH was conducted twice, suspension washing with acetonitrile was conducted, and the resultant solid was dried under reduced pressure, to obtain a compound 12 (6.06 g, quant).

### Synthesis of compound 13

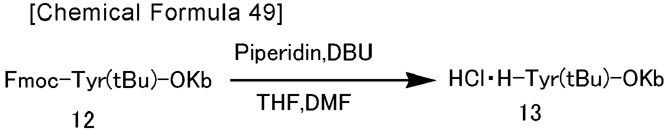

The compound 12 (6.06 g, 5.00 mmol) was dissolved in a mixture of THF (86 ml) and DMF (10 ml), piperidine (743 µL, 7.5 mmol, 1.5 equiv) and DBU (748 µL, 5.00 mmol, 1 equiv) were added and the mixture was stirred at room temperature for 10 minutes. Concentrated hydrochloric acid was added until pH of the reaction mixture became around 6. A solution of saturated saline:water = 2:1 of double amount of the reaction solvent was added to the reaction mixture and the mixture was washed and separated, then, the aqueous layer was discarded, further, washed with saturated saline of double amount of the reaction solvent and the mixture was separated, then, the aqueous layer was discarded. The resultant organic layer was held under reduced pressure and the solvent was distilled off. To the residue was added a mixture of acetonitrile:water = 9:1 and the deposited precipitate was filtrated, and suspended and washed with a mixture of acetonitrile:water = 9:1, further, suspension washing with acetonitrile was conducted, and the resultant solid was dried under reduced pressure, to obtain a compound 13 (4.70 g, 92.9%).

### (Example 21) Synthesis of H-Ser(tBu)-Phe-Arg(Pbf)-Tyr(tBu)-Okb (compound 14)

The following amino acids were introduced into the compound 13 by repeating a 1 pot condensation de-protection method in which an amine scavenger is added and washing with an ammonium chloride aqueous solution is conducted, to obtain a compound 14.
First residue: Fmoc-Arg(Pbf)-OH
Second residue: Fmoc-Phe-OH
Third residue: Fmoc-Ser(tBu)-OH

### (Example 22) Synthesis of H-Asn(Trt)-Ser(tBu)-Phe-Arg(Pbf)-Tyr(tBu)-Okb (compound 15)

The resultant compound 14 (7.58 g, 4.43 mmol) was dissolved in THF so as to give a concentration of 0.05 M, and DIPEA (1.93 ml, 11.1 mmol, 2.5 equiv) was added. Fmoc-Asn(Trt)-OH (3.44 g, 5.76 mmol, 1.3 equiv), COMU (2.28 g, 5.32 mmol, 1.3 equiv) and Oxyma (0.76 g, 5.32 mmol, 1.3 equiv) were dissolved in 8.9 ml of DMF, and DIPEA (1.93 ml, 11.1 mmol, 2.5 equiv) was added and the mixture was stirred at room temperature for 2 minutes. The resultant mixture was added to a THF solution of the compound 14, and the mixture was stirred at room temperature for 10 minutes. As the amine scavenger, propylamine (728 µL, 8.86 mmol, 2 equiv) was added and the mixture was stirred at room temperature for 30 minutes. Piperidine (658 µL, 6.65 mmol, 1.5 equiv) and DBU (4.64 ml, 31.0 mmol, 7 equiv) were added and the mixture was stirred at room temperature for 10 minutes. Concentrated hydrochloric acid was added until pH of the reaction mixture became around 6. A solution of saturated ammonium chloride aqueous solution:water = 1:2 of double amount of the reaction solvent was added to the reaction mixture, and the mixture was washed and separated, and the aqueous layer was discarded. Further, a solution of saturated saline:water = 1:2 of double amount of the reaction solvent was added, and the mixture was washed and separated, and the aqueous layer was discarded. Further, saturated saline of double amount of the reaction solvent was added, and the mixture was washed and separated, and the aqueous layer was discarded. The resultant organic layer was held under reduced pressure and the solvent was distilled off. To the residue was added a mixture of acetonitrile:water = 9:1 and the deposited precipitate was filtrated, further, suspension washing with acetonitrile was conducted, and the resultant solid was dried under reduced pressure, to obtain a compound 15 (8.74 g, 95.4%).

### (Example 23) Synthesis of H-Gly-Leu-Gly-Cys(Trt)-Asn(Trt)-Ser(tBu)-Phe-Arg(Pbf)-Tyr(tBu)-Okb (compound 16)

The following amino acids were introduced into the resultant compound 15 by repeating a 1 pot condensation de-protection method in which an amine scavenger is added and washing with an ammonium chloride aqueous solution is conducted, to obtain a compound 16.
Sixth residue: Fmoc-Cys(Trt)-OH
Seventh residue: Fmoc-Gly-OH
Eighth residue: Fmoc-Leu-OH
Ninth residue: Fmoc-Gly-OH

### (Example 24) Synthesis of H-Gln(Trt)-Ser(ψ_{Me,Me}Pro)-Gly-Leu-Gly-Cys(Trt)-Asn(Trt)-Ser(tBu)-Phe-Arg(Pbf)-Tyr(tBu)-Okb (compound 17)

The compound 16 (10.68 g, 4.06 mmol) obtained in Example 23 was dissolved in a mixture of THF:DMF (9/1) so as to give a concentration of 0.05 M, Fmoc-Gln(Trt)-Ser(ψ_{Me,Me}Pro)-OH (3.88 g, 5.28 mmol, 1.3 equiv), DMT-MM (1.30 g, 4.59 mmol, 1.13 equiv) and DIPEA (845 µL, 4.87 mmol, 1.2 equiv) were added and the mixture was stirred at room temperature for 30 minutes. As the amine scavenger, propylamine (665 µL, 8.12 mmol, 2 equiv) was added and the mixture was stirred at room temperature for 30 minutes. Piperidine (601 µL, 6.09 mmol, 1.5 equiv) and DBU (6.05 ml, 40.6 mmol, 10 equiv) were added and the mixture was stirred at room temperature for 10 minutes. Concentrated hydrochloric acid was added until pH of the reaction mixture became around 6. A solution of saturated ammonium chloride aqueous solution:water = 1:2 of double amount of the reaction solvent was added to the reaction mixture, and the mixture was washed and separated, and the aqueous layer was discarded. Further, a solution of saturated saline:water = 1:2 of double amount of the reaction solvent was added, and the mixture was washed and separated, and the aqueous layer was discarded. Further, saturated saline of double amount of the reaction solvent was added, and the liquid was washed and separated, and the aqueous layer was discarded. The resultant organic layer was held under reduced pressure and the solvent was distilled off. To the residue was added water and the deposited precipitate was filtrated, and suspended and washed with n-hexane, and the resultant solid was dried under reduced pressure, to obtain a compound 17 (12.85 g, 100.0%). The resultant compound 17 was analyzed by TLC (n-hexane:ethyl acetate = 9:1), but the presence of dibenzofulvene was not observed.

### (Example 25) Synthesis of H-D-Arg-Arg-Pro-Hyp-Gly-Thi-Ser-D-Tic-Oic-Arg-OH (compound 21)

### Synthesis of compound 18

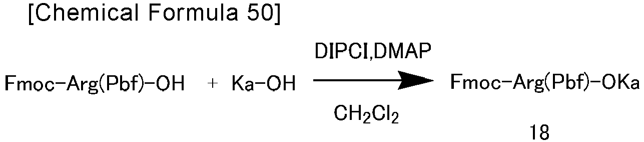

3,4,5-trioctadecyloxybenzyl alcohol (expressed as "Ka-OH")(9.14 g, 10.00 mmol) was dissolved in DCM (100 ml), Fmoc-Arg(Pbf)-OH (9.73 g, 15.00 mmol, 1.5 equiv), DIPCI (2337 µL, 15.00 mmol, 1.5 equiv) and DMAP (61 mg, 0.50 mmol, 0.05 equiv) were added and the mixture was stirred at room temperature for 30 minutes. Propylamine (1644 µL, 20.0 mmol, 2.0 equiv) was added and the mixture was stirred at room temperature for 30 minutes. The precipitate was filtrated, and the filtrate was distilled off under reduced pressure. To the residue was added MeOH and the deposited precipitate was filtrated, suspension washing with MeOH was conducted, further, suspension washing with acetonitrile was conducted, and the resultant solid was dried under reduced pressure, to obtain a compound 18 (15.40 g, 99.7%).

### Synthesis of compound 19

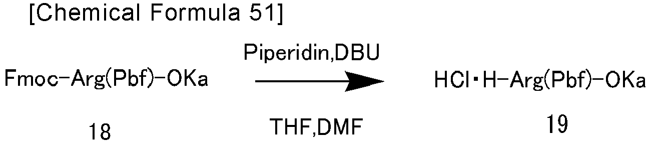

The compound 18 (15.40 g, 10.00 mmol) was dissolved in a mixture of THF (179 ml) and DMF (20 ml), piperidine (1480 µL, 14.9 mmol, 1.5 equiv) and DBU (1490 µL, 9.96 mmol, 1 equiv) were added and the mixture was stirred at room temperature for 10 minutes. Concentrated hydrochloric acid was added until pH of the reaction mixture became around 6. A solution of saturated saline:water = 2:1 of double amount of the reaction solvent was added to the reaction mixture and the mixture was washed and separated, then, the aqueous layer was discarded, further, washed with saturated saline of double amount of the reaction solvent and the mixture was separated, then, the aqueous layer was discarded. The resultant organic layer was held under reduced pressure and the solvent was distilled off. To the residue was added acetonitrile and the deposited precipitate was filtrated, and suspended and washed with acetonitrile, and the resultant solid was dried under reduced pressure, to obtain a compound 19 (14.39 g, quant).

### Synthesis of compound 20 (HCl•H-D-Arg(Pbf)-Arg(Pbf)-Pro-Hyp-Gly-Thi-Ser(tBu)-D-Tic-Oic-Arg(Pbf)-OKa)

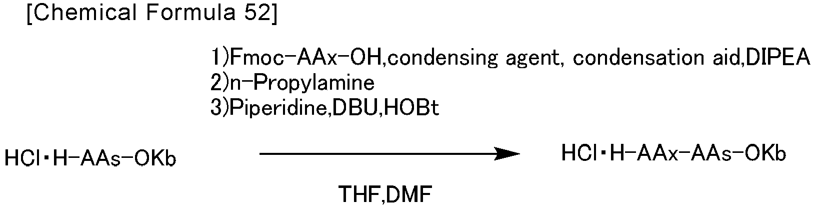

The compound 19 (13.52 g, 9.95 mmol) was subjected to the following method repeatedly, to obtain a compound 20 (24.31 g, 81.6%).

Starting raw materials were dissolved in a mixture of THF:DMF (9/1) so as to give a concentration of 0.05 M, Fmoc-AAx-OH (the equivalent is shown in AA equivalent in the table of the reaction conditions), a condensing agent show in the following table (the equivalent is shown in condensing agent equivalent in the table of the reaction conditions), a condensation aid shown in the following table (the equivalent is shown in condensation aid equivalent in the table of the reaction conditions) and DIPEA (the equivalent is shown in base equivalent in the table of the reaction conditions) were added and the condensation reaction thereof was conducted as shown in the table of the reaction conditions. Next, as the amine scavenger, propylamine (the equivalent is shown in scavenger equivalent in the table of the reaction conditions) was added and the mixture was stirred at room temperature for 30 minutes. Thereafter, HOBt (the equivalent is shown in HOBt equivalent in the table of the reaction conditions), piperidine (1.5 equiv) and DBU (the equivalent is shown in DBU equivalent in the table of the reaction conditions) were added and the mixture was stirred at room temperature for 10 minutes. Next, concentrated hydrochloric acid was added until pH of the reaction mixture became around 6.

The first washing solution shown in the table of the washing conditions was added in double amount of the reaction solvent to the reaction mixture, and the mixture was washed and separated, and the aqueous layer was discarded. If necessary, the second washing solution shown in the table of the washing conditions was added in double amount of the reaction solvent, and the mixture was washed and separated, and the aqueous layer was discarded. Further, saturated saline of double amount of the reaction solvent was added, and the mixture was washed and separated, and the aqueous layer was discarded. The resultant organic layer was held under reduced pressure and the solvent was distilled off. To the residue was added a poor solvent shown in the table of the washing conditions and the deposited precipitate was filtrated, further, a suspension washing solvent shown in the table of the washing conditions was added and suspension washing was conducted, and the resultant solid was dried under reduced pressure, to obtain an amino acid condensate.

**[Table 2]**

| Reaction Consition Table | | | | | | | | | | | |
|---|---|---|---|---|---|---|---|---|---|---|---|
| | Fmoc-AAx-OH | AA equivalent | condensing agent | condensing agent equivalent | condensation aid | condensation aid equivalent | base equivalent | reaction condition | scavenger equivalent | HOBt equivalent | DBU equivalent |
| 2nd residue | Fmoc-Oic-OH | 1.5 | DMT-MM | 1.4 | none | none | 1.2 | R.T. for 30 min | 3 | 1 | 8 |
| 3rd residue | Fmoc-D-Tic-OH | 1.5 | COMU | 1.45 | Oxyma | 1.45 | 4.8 | R.T. for 30 min | 3 | 0 | 7 |
| 4th residue | Fmoc-Ser(tBu)-OH | 1.5 | DMT-MM | 1.4 | none | none | 1.2 | R.T. for 30 min | 3 | 0 | 7 |
| 5th residue | Fmoc-Thi-OH | 1.3 | DMT-MM | 1.2 | none | none | 1.2 | R.T. for 30 min | 2 | 0 | 7 |
| 6th residue | Fmoc-Gly-OH | 1.3 | DMT-MM | 1.2 | none | none | 1.2 | R.T. for 30 min | 2 | 0 | 7 |
| 7th residue | Fmoc-Hyp-OH | 1.3 | DMT-MM | 1.2 | none | none | 1.2 | R.T. for 30 min | 2 | 0 | 7 |
| 8th residue | Fmoc-Pro-OH | 1.5 | DMT-MM | 1.45 | none | none | 1.2 | R.T. for 30 min | 2 | 0 | 7 |
| 9th residue | Fmoc-Arg(Pbf)-OH | 1.5 | DMT-MM | 1.45 | none | none | 1.2 | R.T. for 30 min | 2 | 0 | 7 |
| 10th residue | Fmoc-D-Arg(Pbf)-OH | 1.8 | DMT-MM | 1.62 | none | none | 2.4 | R.T. for 30 min, then 40 °C for 30 min | 5 | 0 | 7 |

**[Table 3]**

| **Washing Condition Table** | | | | |
|---|---|---|---|---|
| | **washing solution (1st)** | **washing solution (2nd)** | **poor solvent** | **suspension-washing solvent** |
| **2nd residue** | **saturated saline:water = 1:2** | **none** | **acetonitrile** | **acetonitrile** |
| **3rd residue** | **saturated ammonium chloride aqueous solution:water = 1:2** | **saturated saline:water = 1:2** | **cold water** | **cold acetonitrile** |
| **4th residue** | **saturated saline:water = 1:2** | **none** | **cold water** | **cold acetonitrile** |
| **5th residue** | **saturated saline:water = 1:2** | **none** | **acetonitrile:water = 9:1** | **acetonitrile** |
| **6th residue** | **saturated saline:water = 1:2** | **none** | **acetonitrile:water = 9:1** | **acetonitrile** |
| **7th residue** | **saturated saline:water = 1:2** | **none** | **acetonitrile:water = 9:1** | **acetonitrile** |
| **8th residue** | **saturated saline:water = 1:2** | **none** | **cold acetonitrile** | **cold acetonitrile** |
| **9th residue** | **saturated saline:water = 1:2** | **none** | **cold acetonitrile** | **cold acetonitrile** |
| **10th residue** | **saturated saline:water = 1:2** | **none** | **acetonitrile** | **acetonitrile** |

### Synthesis of compound 21

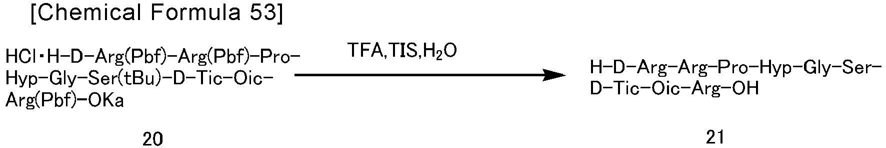

The compound 20 (10.0 g, 3.1 mmol) was dissolved in 155 ml of a mixture of TFA:TIS:water = 90:2.5:7.5, and the mixture was stirred at room temperature for 4 hours. The reaction mixture was filtrated through Celite. The filtration residue was washed with 30 ml of TFA and filtrated. The resultant filtrates were combined together, and put into 1.85 L of cold isopropyl ether while stirring. The resultant solid was filtrated, and the filtration residue was further suspended and washed with cold isopropyl ether and filtrated, twice. The resultant solid was dried, to obtain a compound 21 (5.17 g, Quant, HPLC purity 77.36%). The resultant compound was measured by LC-MS, to observe 652.9 [M+2H⁺]/2.

### (Example 26) Synthesis of H-Ala-Gln(Trt)-Ser(ψ_{Me,Me}Pro)-Gly-Leu-Gly-Cys(Trt)-Asn(Trt)-Ser(tBu)-Phe-Arg(Pbf)-Tyr(tBu)-OKb (compound 22)

Fmoc-Ala-OH was introduced into the compound 17 obtained in Example 24 by a 1 pot condensation de-protection method in which an amine scavenger is added and washing with an ammonium chloride aqueous solution is conducted, to obtain a compound 22 (12.42 g, Quant).

### (Example 27) Synthesis of Fmoc-Ser(tBu)-Leu-Arg(Pbf)-Arg(Pbf)-Ser(tBu)-Ser(tBu)-Cys(Trt)-Phe-Gly-OH (compound 26)

### Synthesis of compound 23 (HCl · H-Gly-OKb)

Fmoc-Gly-OH was used in the same manner as in Example 17 on Kb-OH (7.57 g, 10.0 mmol), to obtain a compound 23 (7.90 g, 93.1%).

### Synthesis of compound 24 (HCl · H-Leu-Arg(Pbf)-Arg(Pbf)-Ser(tBu)-Ser(tBu)-Cys(Trt)-Phe-Gly-OKb)

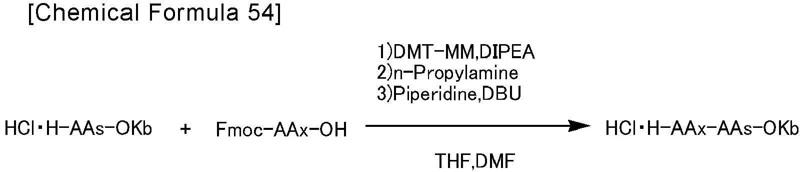

Introduction of
Second residue: Fmoc-Phe-OH
Third residue: Fmoc-Cys(Trt)-OH
Fourth residue: Fmoc-Ser(tBu)-OH
Fifth residue: Fmoc-Ser(tBu)-OH
Sixth residue: Fmoc-Arg(Pbf)-OH
Seventh residue: Fmoc-Arg(Pbf)-OH
Eighth residue: Fmoc-Leu-OH

Amino acids were introduced sequentially into the compound 23 (7.89 g, 9.25 mmol) by repeating the following method, to obtain a compound 24 (20.27 g, 85.5%).

Starting raw materials were processed using a 1 pot condensation de-protection method in which an amine scavenger is added and washing with an ammonium chloride aqueous solution is conducted (here, Fmoc-amino acid (1.3 equiv) and DMT-MM (1.13 equiv) are used, washing with an ammonium chloride aqueous solution is omitted), to obtain an amino acid condensate.

### Synthesis of compound 25 (Fmoc-Ser(tBu)-Leu-Arg(Pbf)-Arg(Pbf)-Ser(tBu)-Ser(tBu)-Cys(Trt)-Phe-Gly-OKb)

Fmoc-Ser(tBu)-OH was introduced into the compound 24 (20.26 g, 7.91 mmol) by an amino acid condensation general synthesis method, to obtain a compound 25 (22.56 g, Quant).

### Synthesis of compound 26 (Fmoc-Ser(tBu)-Leu-Arg(Pbf)-Arg(Pbf)-Ser(tBu)-Ser(tBu)-Cys(Trt)-Phe-Gly-OH)

A Kb protective group general de-protection method was applied on the compound 25 (22.56 g, 7.80 mmol), to obtain a compound 26 (22.56 g, Quant).

### (Example 28) Synthesis of HCl · H-Ser(tBu)-Leu-Arg(Pbf)-Arg(Pbf)-Ser(tBu)-Ser(tBu)-Cys(Trt)-Phe-Gly-Gly-Arg(Pbf)-Met-Asp(OtBu)-Arg(Pbf)-Ile-Gly-Ala-Gln(Trt)-Ser(ψ_{Me,Me}Pro)-Gly-Leu-Gly-Cys(Trt)-Asn(Trt)-Ser(tBu)-Phe-Arg(Pbf)-Tyr(tBu)-OKb (compound 28)

### Synthesis of compound 27 (HCl · H-Gly-Arg(Pbf)-Met-Asp(OtBu)-Arg(Pbf)-Ile-Gly-Ala-Gln(Trt)-Ser(ψ_{Me,Me}Pro)-Gly-Leu-Gly-Cys(Trt)-Asn(Trt)-Ser(tBu)-Phe-Arg(Pbf)-Tyr(tBu)-OKb)

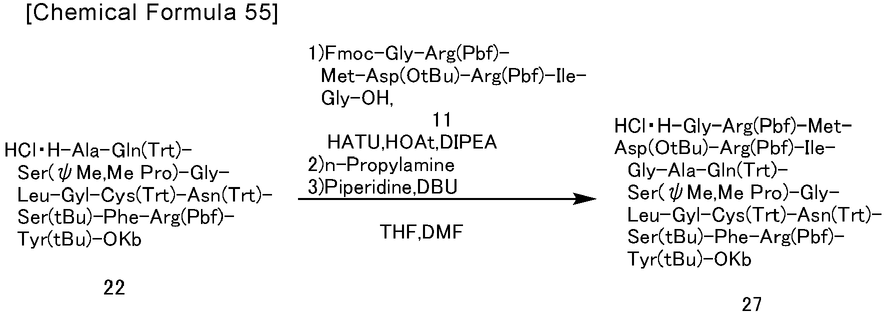

The compound 11 (11.33 g, 5.8 mmol, 1.5 equiv) obtained in Example 19 was dissolved in DMF (38.7 ml), and THF (348 ml) was added for dilution, and the compound 22 (12.42 g, 3.87 mmol, 1.0 equiv) obtained in Example 26, HATU (1.99 g, 5.22 mmol, 1.35 equiv), HOAt (711 mg, 5.22 mmol, 1.35 equiv) and DIPEA (3369 µL, 19.3 mmol, 5.0 equiv) were added, and the mixture was stirred at room temperature for 30 minutes. As the amine scavenger, propylamine (318 µL, 3.87 mmol, 1 equiv) was added and the mixture was stirred at room temperature for 30 minutes. Further, piperidine (575 µL, 5.80 mmol, 1.0 equiv) and DBU (5.79 ml, 38.7 mmol, 10 equiv) were added and the mixture was stirred at room temperature for 10 minutes. Concentrated hydrochloric acid was added until pH of the reaction mixture became around 6. A solution of saturated ammonium chloride aqueous solution:water = 1:2 of double amount of the reaction solvent was added to the reaction mixture, and the mixture was washed and separated, and the aqueous layer was discarded. Further, a solution of saturated saline:water = 1:2 of double amount of the reaction solvent was added, and the mixture was washed and separated, and the aqueous layer was discarded. Further, saturated saline of double amount of the reaction solvent was added, and the liquid was washed and separated, and the aqueous layer was discarded. The resultant organic layer was held under reduced pressure and the solvent was distilled off. To the residue was added a mixture of acetonitrile:water = 9:1 and the deposited precipitate was filtrated, and suspended and washed with acetonitrile, and the resultant solid was dried under reduced pressure, to obtain a compound 27 (15.29 g, quant).

### Synthesis of compound 28 (HCl · H-Ser(tBu)-Leu-Arg(Pbf)-Arg(Pbf)-Ser(tBu)-Ser(tBu)-Cys(Trt)-Phe-Gly-Gly-Arg(Pbf)-Met-Asp(OtBu)-Arg(Pbf)-Ile-Gly-Ala-Gln(Trt)-Ser(ψ_{Me,Me}Pro)-Gly-Leu-Gly-Cys(Trt)-Asn(Trt)-Ser(tBu)-Phe-Arg(Pbf)-Tyr(tBu)-OKb)

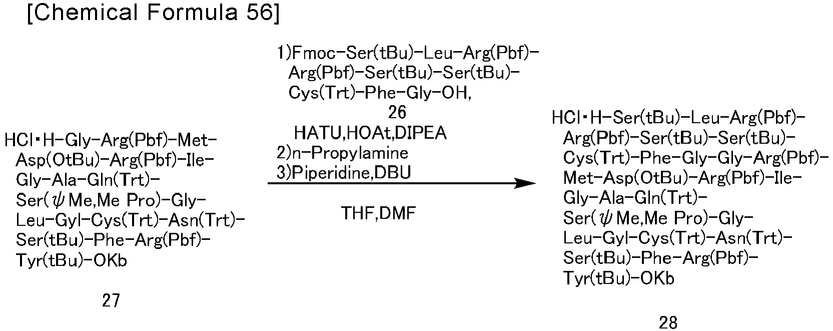

On the compound 27 (17.63 g, 3.87 mmol), the compound 26 (12.47 g, 5.80 mmol, 1.5 equiv) obtained in Example 27 was used and reacted in the same manner as for synthesis of the compound 27, to obtain a compound 28 (26.83 g, quant).

### (Example 29) Synthesis of H-Ser-Leu-Arg-Arg-Ser-Ser-Ser-Phe-Gly-Gly-Arg-Met-Asp-Arg-Ile-Gly-Gln-Ser-Gly-Leu-Gly-Cys-Asn-Ser-Phe-Arg-Tyr-OH (7Cys-23Cys, SS bond) (compound 30)

### Synthesis of compound 29 (H-Ser-Leu-Arg-Arg-Ser-Ser-Ser-Phe-Gly-Gly-Arg-Met-Asp-Arg-Ile-Gly-Gln-Ser-Gly-Leu-Gly-Cys-Asn-Ser-Phe-Arg-Tyr-OH)

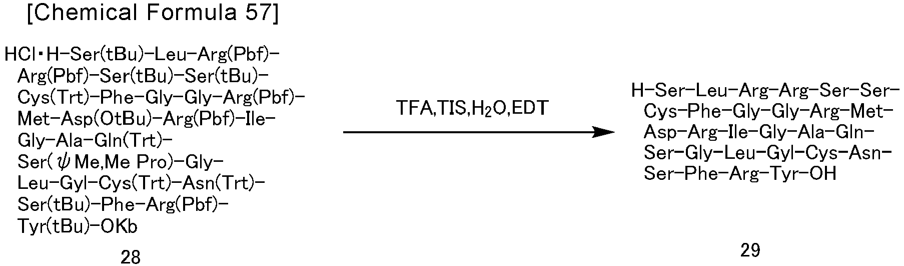

The compound 28 (6.93 g, 1.0 mmol) was dissolved in 200 ml of a mixture of TFA:TIS:water:EDT = 80:5:10:5, and the mixture was stirred at room temperature for 4 hours. The reaction liquid was filtrated through Celite, and the filtration residue was washed with 100 ml of TFA and filtrated. The resultant filtrates were combined together, and put into 3.0 L of cold isopropyl ether while stirring. The resultant solid was filtrated, and the filtration residue was further suspended and washed with cold isopropyl ether and filtrated, twice. The resultant solid was dried, to obtain a compound 29 (3.90 g, Quant, HPLC purity 55.22%).

### Synthesis of compound 30 (H-Ser-Leu-Arg-Arg-Ser-Ser-Ser-Phe-Gly-Gly-Arg-Met-Asp-Arg-Ile-Gly-Gln-Ser-Gly-Leu-Gly-Cys-Asn-Ser-Phe-Arg-Tyr-OH (7Cys-23Cys, SS bond))

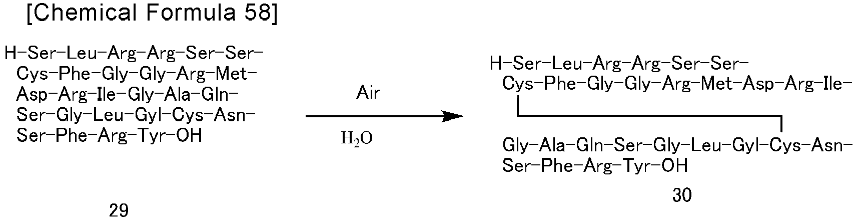

The compound 29 (3.90 g, 1.0 mmol) was dissolved in 3.77 L of water, and the solution was adjusted to pH7.1 with pyridine, and stirred overnight. The resultant reaction mixture was freeze-dried, to obtain a compound 30 (3.47 g, 92.2%, HPLC purity 65.07%). The resultant compound was measured by LC-MS, to observe 1027.2 [M+3H⁺]/3.

### (Example 30) Synthesis of H-Glu(OtBu)-Ile-Pro-Glu(OtBu)-Glu(OtBu)-Tyr(tBu)-Leu-OKb (compound 33)

### Synthesis of compound 31

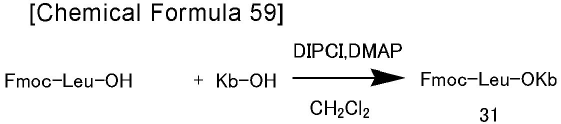

Fmoc-Leu-OH was used in the same manner as in Example 18 on Kb-OH (2.27 g, 3.00 mmol), to obtain a compound 31 (3.26 g, 99.6%).

### Synthesis of compound 32

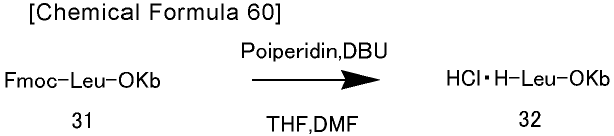

A de-Fmoc general synthesis method was applied on the compound 31 (3.26 g, 2.98 mmol), to obtain a compound 32 (2.72 g, Quant).

### Synthesis of compound 33 (H-Glu(OtBu)-Ile-Pro-Glu(OtBu)-Glu(OtBu)-Tyr(tBu)-Leu-OKb)

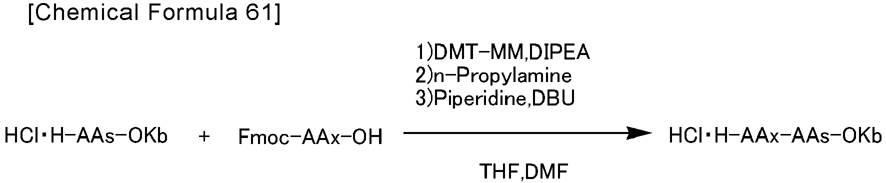

Introduction of
Second residue: Fmoc-Tyr(tBu)-OH
Third residue: Fmoc-Glu(OtBu)-OH
Fourth residue: Fmoc-Glu(OtBu)-OH
Fifth residue: Fmoc-Pro-OH
Sixth residue: Fmoc-Ile-OH
Seventh residue: Fmoc-Glu(OtBu)-OH

Amino acids were introduced sequentially into the compound 32 (2.70 g, 2.98 mmol) by repeating the following method, to obtain a compound 33 (4.40 g, 77.7%).

Starting raw materials were dissolved in a mixture of THF:DMF (9/1) so as to give a concentration of 0.05 M, Fmoc-amino acid (1.3 equiv), DMT-MM (1.2 equiv) and DIPEA (1.2 equiv) were added and the mixture was stirred at room temperature for 30 minutes. As the amine scavenger, propylamine (2 equiv) was added and the mixture was stirred at room temperature for 30 minutes. HOBt (1 equiv)(only second residue), piperidine (1.5 equiv) and DBU (7 equiv, for only second residue: 8 equiv) were added and the mixture was stirred at room temperature for 10 minutes. Concentrated hydrochloric acid was added until pH of the reaction mixture became around 6. A solution of saturated saline:water = 1:2 of double amount of the reaction solvent was added to the reaction mixture, and the mixture was washed and separated, and the aqueous layer was discarded. Further, saturated saline of double amount of the reaction solvent was added, and the mixture was washed and separated, and the aqueous layer was discarded. The resultant organic layer was held under reduced pressure and the solvent was distilled off. To the residue was added acetonitrile and the deposited precipitate was filtrated, and suspended and washed with acetonitrile, and the resultant solid was dried under reduced pressure, to obtain an amino acid condensate.

### (Example 31) Synthesis of Fmoc-Gly-Gly-Asn(Trt)-Gly-Asp(OtBu)-Phe-Glu(OtBu)-OH (compound 38)

### Synthesis of compound 34

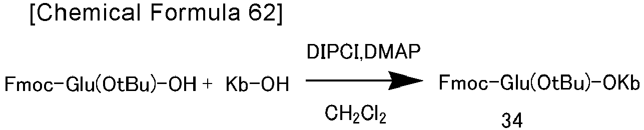

Fmoc-Glu(OtBu)-OH was used in the same manner as in Example 18 on Kb-OH (3.41 g, 4.50 mmol), to obtain a compound 34 (5.05 g, 96.3%).

### Synthesis of compound 35

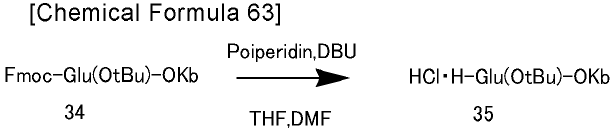

A de-Fmoc general synthesis method was applied on the compound 34 (5.04 g, 4.33 mmol), to obtain a compound 35 (4.24 g, Quant).

### Synthesis of compound 36 (HCl • H-Gly-Asn(Trt)-Gly-Asp(OtBu)-Phe-Glu(OtBu)-OKb)

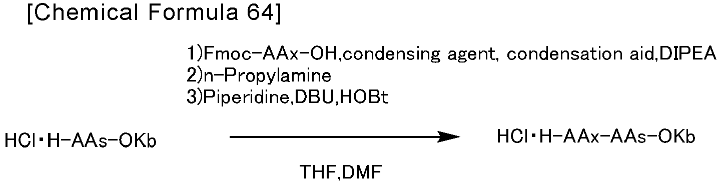

The compound 35 (4.23 g, 4.33 mmol) was processed by the same manner as for synthesis of the compound 20, according to the table of the reaction conditions and the table of the washing conditions shown below, to obtain a compound 36 (7.05 g, 90.8%).

**[Table 4]**

| Reaction Condition Table | | | | | | | | | | | |
|---|---|---|---|---|---|---|---|---|---|---|---|
| | Fmoc-AAx-OH | AA equivalent | condensing agent | condensing agent equivalent | condensation aid | condensation aid equivalent | base equivalent | reaction condition | scavenger equivalent | HOBt equivalent | DBU equivalent |
| 2nd residue | Fmoc-Phe-OH | 1.3 | DMT-MM | 1.2 | none | none | 1.2 | R.T. for 30 min | 2 | 1 | 8 |
| 3rd residue | Fmoc-Asp(OtBu)-OH | 1.3 | DMT-MM | 1.2 | none | none | 1.2 | R.T. for 30 min | 2 | 0 | 7 |
| 4th residue | Fmoc-Gly-OH | 1.3 | DMT-MM | 1.2 | none | none | 1.2 | R.T. for 30 min | 2 | 0 | 7 |
| 5th residue | Fmoc-Asn(Trt)-OH | 1.3 | DMT-MM | 1.2 | none | none | 1.2 | R.T. for 30 min | 2 | 0 | 7 |
| 6th residue | Fmoc-Gly-OH | 1.95 | DMT-MM | 1.8 | none | none | 1.75 | R.T. for 30 min | 2 | 0 | 7 |

**[Table 5]**

| **Washing Condition Table** | | | | |
|---|---|---|---|---|
| | **washing solution (1st)** | **washing solution (2nd)** | **poor solvent** | **suspension-washing solvent** |
| **2nd residue** | **saturated saline:water = 1:2** | **none** | **acetonitrile** | **acetonitrile** |
| **3rd residue** | **saturated saline:water = 1:2** | **none** | **acetonitrile** | **acetonitrile** |
| **4th residue** | **saturated saline:water = 1:2** | **none** | **acetonitrile** | **acetonitrile** |
| **5th residue** | **saturated saline:water** = **1:2** | **none** | **acetonitrile** | **acetonitrile** |
| **6th residue** | **saturated saline:water** = **1:2** | **none** | **acetonitrile** | **acetonitrile** |

### Synthesis of compound 37

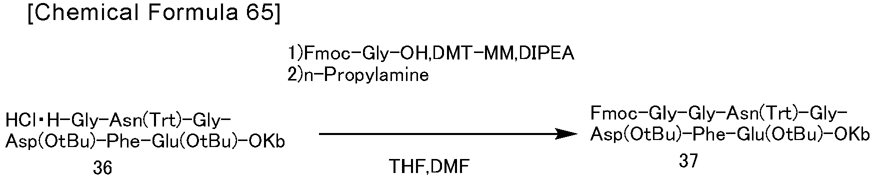

The compound 36 (7.04 g, 3.90 mmol) was dissolved in a mixture of THF (65 ml) and DMF (7.2 ml), Fmoc-Gly-OH (1.39 g, 4.68 mmol, 1.2 equiv), DMT-MM (1.20 g, 4.32 mmol, 1.11 equiv) and DIPEA (753 µL, 4.32 mmol, 1.11 equiv) were added and the mixture was stirred at room temperature for 30 minutes. As the amine scavenger, propylamine (888 µL, 10.8 mmol, 2.77 equiv) was added and the mixture was stirred at room temperature for 30 minutes. The resultant organic layer was held under reduced pressure and the solvent was distilled off. To the residue was added a solution of acetonitrile:water = 9:1 and the solution was cooled, and the deposited precipitate was filtrated, and suspended and washed with acetonitrile, further, suspension washing with acetonitrile was conducted, and the resultant solid was dried under reduced pressure, to obtain a compound 37 (7.16 g, 90.4%).

### Synthesis of compound 38 (Fmoc-Gly-Gly-Asn(Trt)-Gly-Asp(OtBu)-Phe-Glu(OtBu)-OH)

A Kb protective group general de-protection method was applied on the compound 37 (2.20 g, 1.09 mmol), to obtain a compound 38 (1.21 g, 83.0%).

### (Example 32) Synthesis of Fmoc-D-Phe-Pro-Arg(Pbf)-Pro-Gly-Gly-OH (compound 42)

### Synthesis of compound 39 (HCI • H-Gly-OKb)

Fmoc-Gly-OH was used in the same manner as in Example 17 on Kb-OH (3.41 g, 4.5 mmol), to obtain a compound 39 (3.58 g, 90.2%).

### Synthesis of compound 40 (HCl•H-Pro-Arg(Pbf)-Pro-Gly-Gly-OKb)

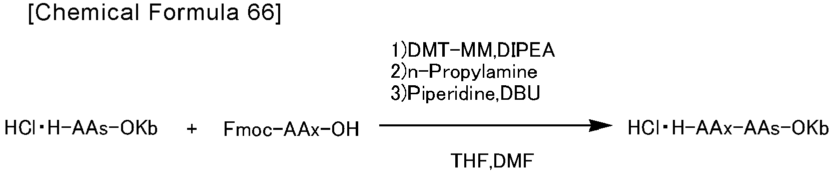

Introduction of
Second residue: Fmoc-Gly-OH
Third residue: Fmoc-Pro-OH
Fourth residue: Fmoc-Arg(Pbf)-OH
Fifth residue: Fmoc-Pro-OH

Amino acids were introduced sequentially into the compound 39 (3.33 g, 3.91 mmol) by repeating the following method, to obtain a compound 40 (5.11 g, 85.7%).

Starting raw materials were dissolved in a mixture liquid of THF:DMF (9/1) so as to give a concentration of 0.05 M, Fmoc-amino acid (1.3 equiv), DMT-MM (1.2 equiv) and DIPEA (1.2 equiv) were added and the mixture was stirred at room temperature for 30 minutes. As the amine scavenger, propylamine (2 equiv) was added and the mixture was stirred at room temperature for 30 minutes. HOBt (1 equiv), piperidine (1.5 equiv) and DBU (7 equiv, only for second residue: 8 equiv) were added and the mixture was stirred at room temperature for 10 minutes. Concentrated hydrochloric acid was added until pH of the reaction mixture became around 6. A solution of saturated saline:water = 1:2 of double amount of the reaction solvent was added to the reaction mixture, and the mixture was washed and separated, and the aqueous layer was discarded. Further, saturated saline of double amount of the reaction solvent was added, and the mixture was washed and separated, and the aqueous layer was discarded. The resultant organic layer was held under reduced pressure and the solvent was distilled off. To the residue was added acetonitrile and the deposited precipitate was filtrated, and suspended and washed with acetonitrile, and the resultant solid was dried under reduced pressure, to obtain an amino acid condensate.

### Synthesis of compound 41

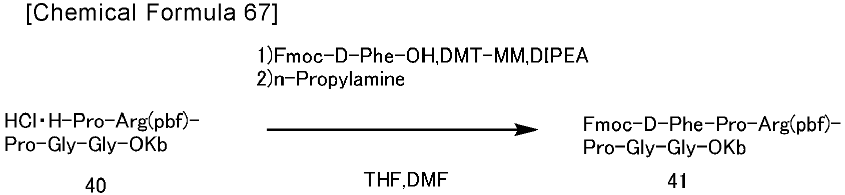

The compound 40 (5.10 g, 3.38 mmol) was dissolved in a mixture of THF (61 ml) and DMF (6.7 ml), Fmoc-D-Phe-OH (1.70 g, 4.39 mmol, 1.3 equiv), DMT-MM (1.12 g, 4.05 mmol, 1.2 equiv) and DIPEA (705 µL, 4.05 mmol, 1.2 equiv) were added and the mixture was stirred at room temperature for 30 minutes. As the amine scavenger, propylamine (555 µL, 6.75 mmol, 2 equiv) was added and the mixture was stirred at room temperature for 30 minutes. The resultant organic layer was held under reduced pressure and the solvent was distilled off. To the residue was added a solution of acetonitrile:water = 9:1 and the solution was cooled, and the deposited precipitate was filtrated, and suspended and washed with acetonitrile, further, suspension washing with acetonitrile was conducted, and the resultant solid was dried under reduced pressure, to obtain a compound 41(5.92 g, 95.2%).

### Synthesis of compound 42 (Fmoc-D-Phe-Pro-Arg(Pbf)-Pro-Gly-Gly-OH)

A Kb protective group general de-protection method was applied on the compound 41 (1.84 g, 1.0 mmol), to obtain a compound 42 (0.942 g, 85.3%).

### (Example 33) Synthesis of H-D-Phe-Pro-Arg(Pbf)-Pro-Gly-Gly-Gly-Gly-Asn(Trt)-Gly-Asp(OtBu)-Phe-Glu(OtBu)-Glu(OtBu)-Ile-Pro-Glu(OtBu)-Glu(OtBu)-Tyr(tBu)-Leu-OKb (compound 44)

### Synthesis of compound 43(HCl • H-Gly-Gly-Asn(Trt)-Gly-Asp(OtBu)-Phe-Glu(OtBu)-Glu(OtBu)-Ile-Pro-Glu(OtBu)-Glu(OtBu)-Tyr(tBu)-Leu-OKb)

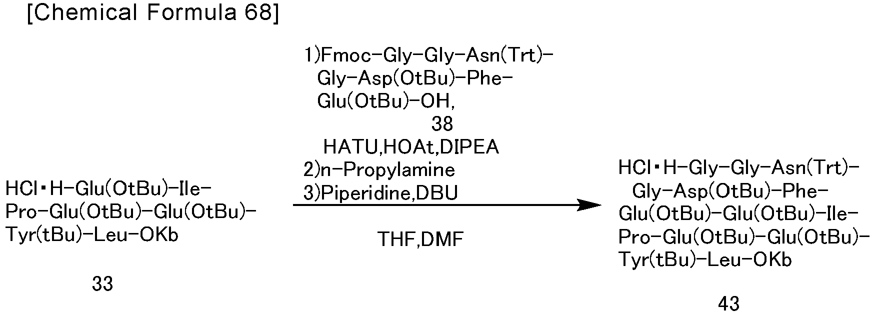

On the compound 33 (568 mg, 0.3 mmol) obtained in Example 30, the compound 38 (573 mg, 0.45 mmol, 1.5 equiv) obtained in Example 31 was used and reacted in the same manner as for synthesis of the compound 27, and liquid-liquid washed, and to the concentrated residue was added a mixture of acetonitrile:water = 9:1 and the deposited precipitate was centrifugally separated, and suspended and washed with methanol, further, suspension washing with acetonitrile was conducted, and the resultant solid was dried under reduced pressure, to obtain a compound 43 (725 mg, 82.6%).

### Synthesis of compound 44 (HCI • H-D-Phe-Pro-Arg(Pbf)-Pro-Gly-Gly-Gly-Gly-Asn(Trt)-Gly-Asp(OtBu)-Phe-Glu(OtBu)-Glu(OtBu)-Ile-Pro-Glu(OtBu)-Glu(OtBu)-Tyr(tBu)-Leu-OKb)

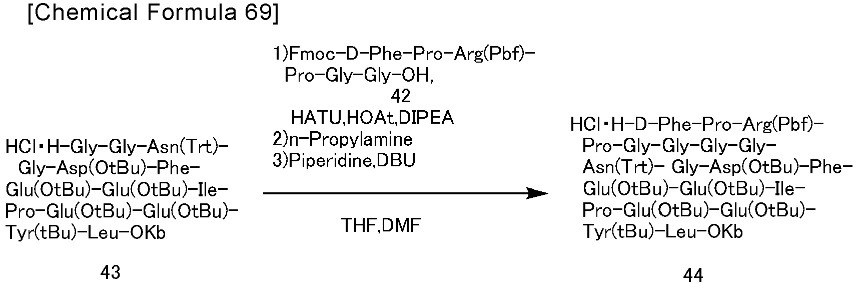

On the compound 43 (715 mg, 0.245 mmol), the compound 42 (405 mg, 0.33 mmol, 1.5 equiv) obtained in Example 32 was used and operated in the same manner as for synthesis of the compound 32, to obtain a compound 44 (863 mg, 93.2%).

### (Example 34) Synthesis of H-D-Phe-Pro-Arg-Pro-Gly-Gly-Gly-Gly-Asn-Gly-Asp-Phe-Glu-Glu-Ile-Pro-Glu-Glu-Tyr-Leu-OH (compound 45)

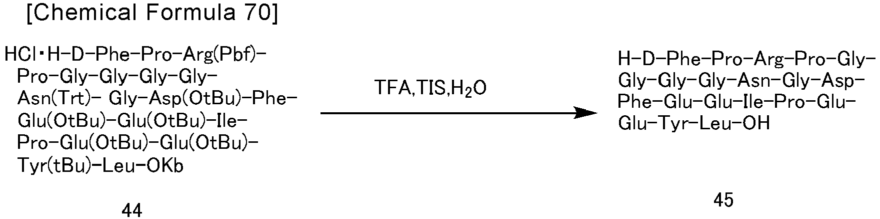

The compound 44 (300 mg, 0.079 mmol) was dissolved in 8 ml of a mixture of TFA:TIS:water = 95:2.5:2.5, and the mixture was stirred at room temperature for 3 hours. The reaction mixture was filtrated through Celite. The filtration residue was washed with 3 ml of TFA and filtrated. The resultant filtrates were combined together, and put into 120 mL of cold isopropyl ether while stirring. The resultant solid was centrifugally separated, and the filtration residue was further suspended and washed with cold isopropyl ether and centrifugally separated, four times. The resultant solid was dried, to obtain a compound 45 (147.7 mg, 77.4%, HPLC purity 74.49%).

### (Example 35) Synthesis of H-Lys(Boc)-Pro-Pro-Ala-Lys(Boc)-Leu-Gln(Trt)-Pro-Arg(Pbf)-OKb (compound 50)

### Synthesis of compound 46

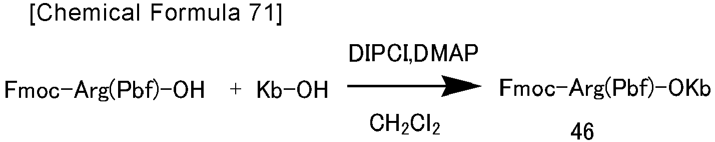

On Kb-OH (2.27 g, 3.0 mmol), Fmoc-Arg(Pbf)-OH (2.92 g, 4.5 mmol, 1.5 equiv) was used in the same manner as in Example 17, to obtain a compound 46 (4.20 g, Quant).

### Synthesis of compound 47

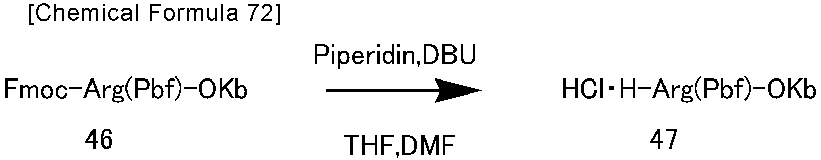

A de-Fmoc general synthesis method was applied on the compound 46 (4.15 g, 2.99 mmol), to obtain a compound 47 (3.51 g, 97.6%).

### Synthesis of compound 48

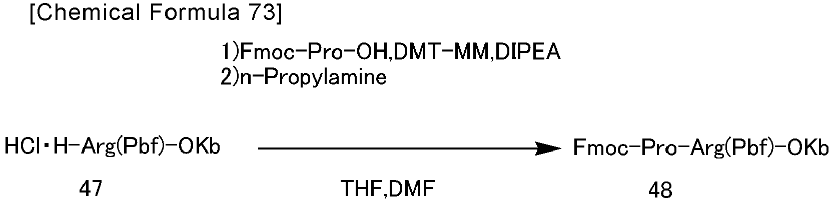

The compound 47 (3.5 g, 2.91 mmol) was dissolved in a mixture of THF (52 ml) and DMF (5.8 ml), Fmoc-Pro-OH (1.28 g, 3.78 mmol, 1.3 equiv), DMT-MM (943 mg, 3.41 mmol, 1.17 equiv) and DIPEA (913 µL, 5.24 mmol, 1.8 equiv) were added and the mixture was stirred at room temperature for 30 minutes. As the amine scavenger, propylamine (479 µL, 5.82 mmol, 2 equiv) was added and the mixture was stirred at room temperature for 30 minutes. The resultant reaction mixture was held under reduced pressure and the solvent was distilled off. To the residue was added acetonitrile and the deposited precipitate was filtrated, and suspended and washed with acetonitrile, and the resultant solid was dried under reduced pressure, to obtain a compound 48 (4.24 g, 98.0%).

### Synthesis of compound 49

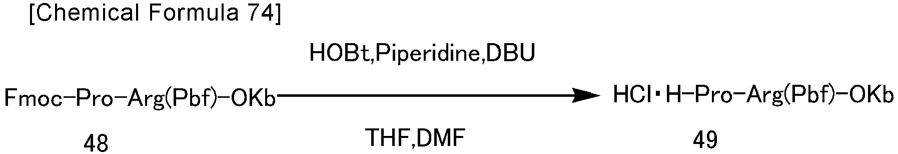

The compound 48 (4.23 g, 2.85 mmol) was dissolved in a mixture liquid of THF (51 ml) and DMF (6 ml), HOBt (388 mg, 2.85 mmol, 1.0 equiv), piperidine (423 µL, 4.27 mmol, 1.5 equiv) and DBU (852 µL, 5.69 mmol, 2 equiv) were added and the mixture was stirred at room temperature for 10 minutes. Concentrated hydrochloric acid was added until pH of the reaction mixture became around 6. A solution of saturated ammonium chloride aqueous solution:water = 1:2 of double amount of the reaction solvent was added to the reaction mixture, and the mixture was washed and separated, and the aqueous layer was discarded. Further, a solution of saturated saline:water = 1:2 of double amount of the reaction solvent was added, and the mixture was washed and separated, and the aqueous layer was discarded. Further, saturated saline of double amount of the reaction solvent was added, and the mixture was washed and separated, and the aqueous layer was discarded, and the solvent was distilled off under reduced pressure. To the residue was added acetonitrile and the deposited precipitate was filtrated, further, suspended and washed with acetonitrile, and the resultant solid was dried under reduced pressure, to obtain a compound 49 (3.75 g, Quant).

### Synthesis of compound 50 (H-Lys(Boc)-Pro-Pro-Ala-Lys(Boc)-Leu-Gln(Trt)-Pro-Arg(Pbf)-OKb)

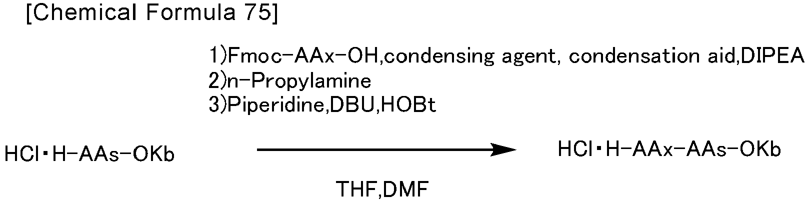

The compound 49 (3.69 g, 2.84 mmol) was processed in the same manner as for synthesis of the compound 20, according to the reaction conditions and the washing conditions shown in the tables below, to obtain a compound 50 (6.02 g, 79.9%).

**[Table 6]**

| **Reaction Consition Table** | | | | | | | | | | | |
|---|---|---|---|---|---|---|---|---|---|---|---|
| | Fmoc-AAx-OH | AA equivalent | condensing agent | condensing agent equivalent | condensation aid | condensation aid equivalent | base equivalent | reaction condition | scavenger equivalent | HOBt equivalent | DBU equivalent |
| 3rd residue | Fmoc-Gln(Trt)-OH | 1.5 | DMT-MM | 1.4 | none | none | 1.4 | R.T. for 30 min | 3 | 0 | 7 |
| 4th residue | Fmoc-Leu-OH | 1.95 | DMT-MM | 1.76 | none | none | 2.4 | R.T. for 30 min | 6 | 0 | 7 |
| 5th residue | Fmoc-Lys(Boc)-OH | 1.3 | DMT-MM | 1.17 | none | none | 1.2 | R.T. for 30 min | 2 | 0 | 10 |
| 6th residue | Fmoc-Ala-OH | 1.3 | DMT-MM | 1.2 | none | none | 1.2 | R.T. for 30 min | 2 | 0 | 7 |
| 7th residue | Fmoc-Pro-OH | 1.3 | DMT-MM | 1.2 | none | none | 1.2 | R.T. for 30 min | 2 | 0 | 7 |
| 8th residue | Fmoc-Pro-OH | 1.3 | DMT-MM | 1.2 | none | none | 1.2 | R.T. for 30 min | 2 | 0 | 7 |
| 9th residue | Fmoc-Arg(Pbf)-OH | 1.5 | DMT-MM | 1.45 | none | none | 1.2 | R.T. for 30 min | 2 | 0 | 7 |

**[Table 7]**

| Washing Condition Table | | | | |
|---|---|---|---|---|
| | washing solution (1st) | washing solution (2nd) | poor solvent | suspension-washing solvent |
| 3rd residue | saturated ammonium chloride aqueous solution:water = 1:2 | saturated saline:water = 1:2 | cold water | cold acetonitrile |
| 4th residue | saturated ammonium chloride aqueous solution:water = 1:2 | saturated saline:water = 1:2 | cold water | cold acetonitrile |
| 5th residue | saturated ammonium chloride aqueous solution:water = 1:2 | saturated saline:water = 1:2 | cold water | cold acetonitrile |
| 6th residue | saturated ammonium chloride aqueous solution:water = 1:2 | saturated saline:water = 1:2 | acetonitrile | acetonitrile |
| 7th residue | saturated ammonium chloride aqueous solution:water = 1:2 | saturated saline:water = 1:2 | acetonitrile | acetonitrile |
| 8th residue | saturated ammonium chloride aqueous solution:water = 1:2 | saturated saline:water = 1:2 | acetonitrile | acetonitrile |
| 9th residue | saturated ammonium chloride aqueous solution:water = 1:2 | saturated saline:water = 1:2 | acetonitrile | acetonitrile |

### (Example 36) Synthesis of Fmoc-Lys(Boc)-Leu-Gln(Trt)-Gln(Trt)-Arg(pbf)-Lys(Boc)-Glu(OtBu)-Ser(tBu)-Lys(Boc)-OH (compound 55)

### Synthesis of compound 51

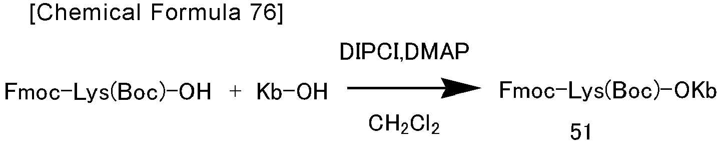

On Kb-OH (1.52 g, 2.0 mmol), Fmoc-Lys(Boc)-OH was used in the same manner as in Example 18, to obtain a compound 51 (2.48 g, Quant).

### Synthesis of compound 52

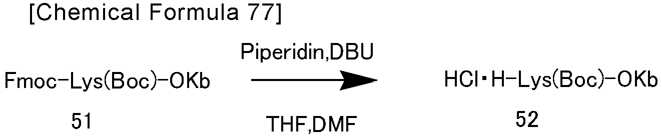

A de-Fmoc general synthesis method was applied on the compound 51 (2.41 g, 1.99 mmol), to obtain a compound 52 (2.01 g, 98.9%).

### Synthesis of compound 53 (HCI • H-Leu-Gln(Trt)-Gln(Trt)-Arg(pbf)-Lys(Boc)-Glu(OtBu)-Ser(tBu)-Lys(Boc)-OKb)

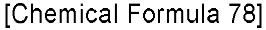

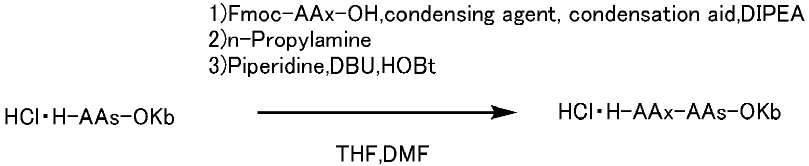

The compound 52 (2.01 g, 1.96 mmol) was processed in the same manner as for synthesis of the compound 20, according to the table of the reaction conditions and the table of the washing conditions shown below, to obtain a compound 53 (4.38 g, 78.2%).

**[Table 8]**

| Reaction Consition Table | | | | | | | | | | | |
|---|---|---|---|---|---|---|---|---|---|---|---|
| | Fmoc-AAx-OH | AA equivalent | condensing agent | condensing agent equivalent | condensati on aid | condensation aid equivalent | base equivalent | reaction condition | scavenger equivalent | HOBt equivalent | DBU equivalent |
| 2nd residue | Fmoc-Ser(tBu)-OH | 1.3 | DMT-MM | 1.17 | none | none | 1.8 | R.T. for 30 min | 2 | 0 | 7 |
| 3rd residue | Fmoc-Glu(OtBu)-OH | 1.3 | DMT-MM | 1.17 | none | none | 1.8 | R.T. for 30 min | 2 | 0 | 10 |
| 4th residue | Fmoc-Lys(Boc)-OH | 1.3 | DMT-MM | 1.17 | none | none | 1.8 | R.T. for 30 min | 2 | 0 | 10 |
| 5th residue | Fmoc-Arg(Pbf)-OH | 1.3 | DMT-MM | 1.17 | none | none | 1.8 | R.T. for 30 min | 2 | 0 | 7 |
| 6th residue | Fmoc-Gln(Trt)-OH | 1.3 | DMT-MM | 1.17 | none | none | 1.8 | R.T. for 30 min | 2 | 0 | 10 |
| 7th residue | Fmoc-Gln(Trt)-OH | 1.3 | DMT-MM | 1.17 | none | none | 1.8 | R.T. for 30 min | 2 | 0 | 10 |
| 8th residue | Fmoc-Leu-OH | 1.3 | DMT-MM | 1.17 | none | none | 1.8 | R.T. for 30 min | 2 | 0 | 7 |

**[Table 9]**

| Washing Condition Table | | | | |
|---|---|---|---|---|
| | washing solution (1st) | washing solution (2nd) | poor solvent | suspension-washing solvent |
| 2nd residue | saturated ammonium chloride aqueous solutlon:water = 1:2 | saturated sallne:water = 1:2 | acetonitrile | acetonitrile |
| 3rd residue | saturated ammonium chloride aqueous solutlon:water = 1:2 | saturated sallne:water = 1:2 | acetonitrile | acetonitrile |
| 4th residue | saturated ammonium chloride aqueous solution:water = 1:2 | saturated sallne:water = 1:2 | acetonitrile | acetonitrile |
| 5th residue | saturated ammonium chloride aqueous solutlon:water = 1:2 | saturated saline:water = 1:2 | acetonitrile | acetonitrile |
| 6th residue | saturated ammonium chloride aqueous solution:water = 1:2 | saturated sallne:water = 1:2 | acetonitrile | acetonitrile |
| 7th residue | saturated ammonium chloride aqueous solution:water = 1:2 | saturated saline:water = 1:2 | acetonitrile | acetonitrile |
| 8th residue | saturated ammonium chloride aqueous solution:water = 1:2 | saturated sallne:water = 1:2 | acetonitrile | acetonitrile |

### Synthesis of compound 54

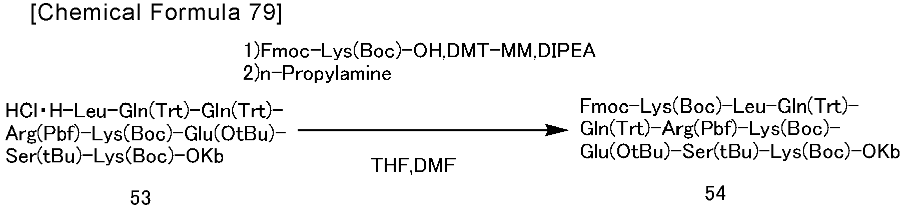

The compound 53 (4.36 g, 1.53 mmol) was operated in the same manner as for the compound 48 using Fmoc-Lys(Boc)-OH (934 mg, 1.99 mmol, 1.3 equiv) and DIPEA (481 µL, 2.76 mmol, 1.8 equiv), to obtain a compound 54 (5.11 g, Quant).

### Synthesis of compound 55 (Fmoc-Lys(Boc)-Leu-Gln(Trt)-Gln(Trt)-Arg(pbf)-Lys(Boc)-Glu(OtBu)-Ser(tBu)-Lys(Boc)-OH)

A Kb protective group general de-protection method was applied on the compound 54 (2.55 g, 0.764 mmol), to obtain a compound 55 (2.26 g, Quant).

### (Example 37) Synthesis of Boc-Gly-Ser(tBu)-Ser(n-octanoyl)-Phe-Leu-Ser(tBu)-Pro-Glu(OtBu)-His(Trt)-Gln(Trt)-OH (compound 61)

### Synthesis of compound 56

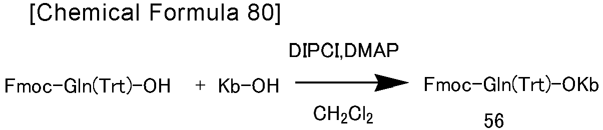

On Kb-OH (2.27 g, 3.0 mmol), Fmoc-Gln(Trt)-OH was used in the same manner as in Example 18, to obtain a compound 56 (4.21 g, Quant).

### Synthesis of compound 57

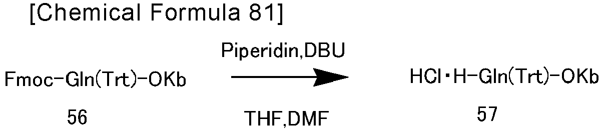

A de-Fmoc general synthesis method was applied on the compound 56 (4.04 g, 2.99 mmol), to obtain a compound 57 (3.62 g, Quant).

### Synthesis of compound 58 (HCI • H-Ser(tBu)-Ser-Phe-Leu-Ser(tBu)-Pro-Glu(OtBu)-His(Trf)-Gln(Trt)-OKb)

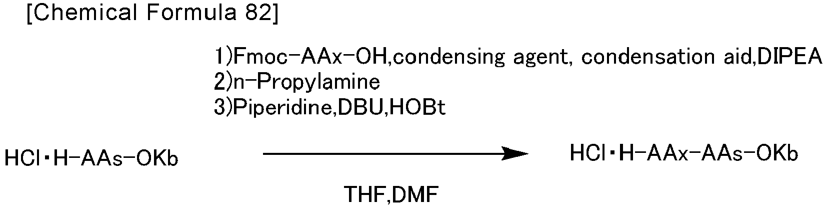

The compound 57 (3.48 g, 2.99 mmol) was processed in the same manner as for synthesis of the compound 20, according to the reaction conditions and washing conditions in the tables below, to obtain a compound 58 (4.60 g, 72.1%).

**[Table 10]**

| Reaction Consition Table | | | | | | | | | | | |
|---|---|---|---|---|---|---|---|---|---|---|---|
| | Fmoc-AAx-OH | AA equivalent | condensing agent | condensing agent equivalent | condensation aid | condensation aid equivalent | base equivalent | reaction condition | scavenger equivalent | HOBt equivalent | DBU equivalent |
| 2nd residue | Fmoc-His(Trt)-OH | 1.3 | DMT-MM | 1.17 | none | none | 3.6 | R.T. for 30 min | 2 | 1 | 8 |
| 3rd residue | Fmoc-Glu(OtBu)-OH | 1.3 | DMT-MM | 1.17 | none | none | 3.6 | R.T. for 30 min | 2 | 0 | 7 |
| 4th residue | Fmoc-Pro-OH | 1.3 | DMT-MM | 1.17 | none | none | 3.6 | R.T. for 30 min | 2 | 0 | 7 |
| 5th residue | Fmoc-Ser(Pbf)-OH | 1.5 | DMT-MM | 1.45 | none | none | 3.6 | R.T. for 30 min | 3 | 0 | 10 |
| 6th residue | Fmoc-Leu-OH | 1.95 | DMT-MM | 1.76 | none | none | 3.6 | R.T. for 30 min | 6 | 0 | 7 |
| 7th residue | Fmoc-Phe-OH | 1.3 | DMT-MM | 1.17 | none | none | 3.6 | R.T. for 30 min | 2 | 0 | 10 |
| 8th residue | Fmoc-Ser-OH | 1.3 | DMT-MM | 1.17 | none | none | 3.6 | R.T. for 30 min | 2 | 0 | 7 |
| 9th residue | Fmoc-Ser(tBu)-OH | 1.3 | DMT-MM | 1.17 | none | none | 3.6 | R.T. for 30 min | 2 | 0 | 10 |

**[Table 11]**

| | | | | |
|---|---|---|---|---|
| Washing Condition Table | | | | |

| | washing solution (1st) | washing solution (2nd) | poor solvent | suspension-washing solvent |
|---|---|---|---|---|
| 2nd residue | saturated ammonium chloride aqueous solution:water = 1:2 | saturated saline:water = 1:2 | acetonitrile | acetonitrile |
| 3rd residue | saturated ammonium chloride aqueous solution:water = 1:2 | saturated saline:water = 1:2 | acetonitrile | acetonitrile |
| 4th residue | saturated ammonium chloride aqueous solution:water = 1:2 | saturated saline:water = 1:2 | no solidification | no suspension-washing |
| 5th residue | saturated ammonium chloride aqueous solution:water = 1:2 | saturated saline:water = 1:2 | no solidification | no suspension-washing |
| 6th residue | saturated ammonium chloride aqueous solution:water = 1:2 | saturated saline:water = 1:2 | acetonitrile | acetonitrile |
| 7th residue | saturated ammonium chloride aqueous solution:water = 1:2 | saturated saline:water = 1:2 | cold acetonitrile:water = 9:1 | acetonitrile |
| 8th residue | saturated ammonium chloride aqueous solution:water = 1:2 | saturated saline:water = 1:2 | acetonitrile | acetonitrile |
| 9th residue | saturated ammonium chloride aqueous solution:water = 1:2 | saturated saline:water = 1:2 | cold acetonitrile | cold acetonitrile |

### Synthesis of compound 59

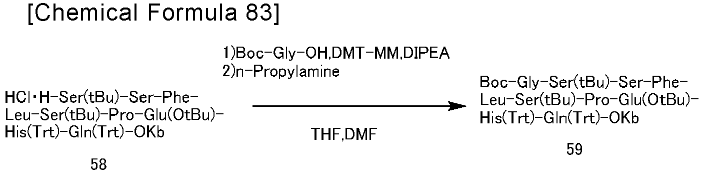

The compound 58 (4.59 g, 1.87 mmol) was operated in the same manner as for the compound 48 using Boc-Gly-OH (425 mg, 2.43 mmol, 1.3 equiv) and DIPEA (1170 µL, 6.72 mmol, 3.6 equiv), to obtain a compound 59 (4.55 g, 94.5%).

### Synthesis of compound 60

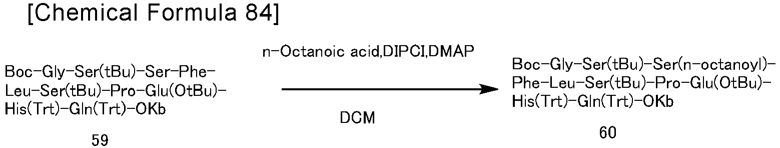

The compound 59 (4.55 g, 1.76 mmol) was dissolved in DCM (35 ml), caprylic acid (628 mg, 3.96 mmol, 2.25 equiv), WSC•HCl (760 mg, 3.96 mmol, 2.2 g equiv) and DMAP (11 mg, 0.088 mmol, 0.05 equiv) were added and the mixture was stirred at room temperature for 30 minutes and at 40°C for 1 hour. As the amine scavenger, propylamine (1086 µL, 13.2 mmol, 7.5 equiv) was added and the mixture was stirred at room temperature for 30 minutes. A solution of saturated ammonium chloride aqueous solution:water = 1:2 of double amount of the reaction solvent was added to the reaction mixture, and the mixture was washed and separated, and the aqueous layer was discarded. Further, a solution of saturated saline:water = 1:2 of double amount of the reaction solvent was added, and the mixture was washed and separated, and the aqueous layer was discarded. Further, saturated saline of double amount of the reaction solvent was added, and the mixture was washed and separated, and the aqueous layer was discarded. The resultant organic layer was held under reduced pressure and the solvent was distilled off. To the residue was added acetonitrile, and the deposited precipitate was filtrated, and suspended and washed with acetonitrile, and the resultant solid was dried under reduced pressure, to obtain a compound 60 (4.48 g, 94.1%).

### Synthesis of compound 61 (Boc-Gly-Ser(tBu)-Ser(n-Octanoyl)-Phe-Leu-Ser(tBu)-Pro-Glu(OtBu)-His(Trt)-Gln(Trt)-OH)

A Kb protective group general de-protection method was applied on the compound 60 (4.47 g, 1.65 mmol), to obtain a compound 61 (3.26 g, Quant).

### (Example 38) Synthesis of Boc-Gly-Ser(tBu)-Ser(n-Octanoyl)-Phe-Leu-Ser(tBu)-Pro-Glu(OtBu)-His(Trt)-Gln(Trt)-Lys(Boc)-Leu-Gln(Trt)-Gln(Trt)-Arg(Pbf)-Lys(Boc)-Glu(OtBu)-Ser(tBu)-Lys(Boc)-Lys(Boc)-Pro-Pro-Ala-Lys(Boc)-Leu-Gln(Trt)-Pro-Arg(Pbf)-OKb (compound 63)

### Synthesis of compound 62(HCl•H-Lys(Boc)-Leu-Gln(Trt)-Gln(Trt)-Arg(Pbf)-Lys(Boc)-Glu(OtBu)-Ser(tBu)-Lys(Boc)-Lys(Boc)-Pro-Pro-Ala-Lys(Boc)-Leu-Gln(Trt)-Pro-Arg(Pbf)-OKb)

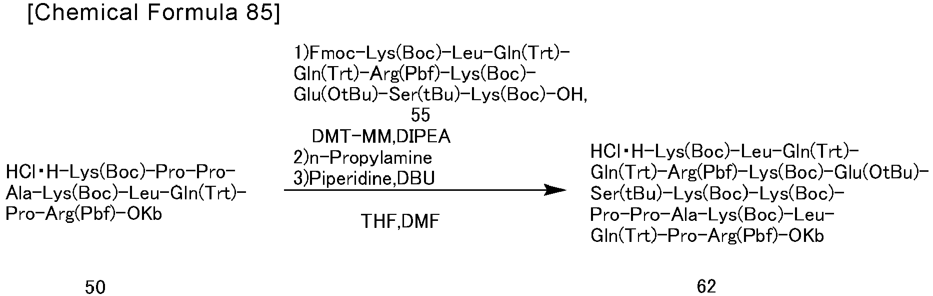

The compound 50 (251 mg, 0.1 mmol) obtained in Example 35 was dissolved in a mixture of THF (9 ml) and DMF (1.0 ml), the compound 61 (443 mg, 0.15 mmol, 1.5 equiv) obtained in Example 36, DMT-MM (40.0 mg, 0.405 mmol, 1.45 equiv) and DIPEA (63 µL, 0.36 mmol, 3.6 equiv) were added and the mixture was stirred at room temperature for 30 minutes. As the amine scavenger, propylamine (25.0 µL, 0.3 mmol, 3 equiv) was added and the mixture was stirred at room temperature for 30 minutes. Piperidine (15 µL, 0.45 mmol, 1.5 equiv) and DBU (150 µL, 3.0 mmol, 10 equiv) were added and the mixture was stirred at room temperature for 10 minutes. Concentrated hydrochloric acid was added until pH of the reaction mixture became around 6. A solution of saturated saline:water = 1:2 of double amount of the reaction solvent was added to the reaction mixture, and the mixture was washed and separated, and the aqueous layer was discarded. Further, saturated saline of double amount of the reaction solvent was added, and the mixture was washed and separated, and the aqueous layer was discarded. The resultant organic layer was held under reduced pressure and the solvent was distilled off. To the residue was added cold acetonitrile and the deposited precipitate was filtrated, further, suspended and washed with cold acetonitrile, and the resultant solid was dried under reduced pressure, to obtain a compound 62 (485 mg, Quant).

### Synthesis of compound 63 (Boc-Gly-Ser(tBu)-Ser(n-Octanoyl)-Phe-Leu-Ser(tBu)-Pro-Glu(OtBu)-His(Trt)-Gln(Trt)-Lys(Boc)-Leu-Gln(Trt)-Gln(Trt)-Arg(Pbf)-Lys(Boc)-Glu(OtBu)-Ser(tBu)-Lys(Boc)-Lys(Boc)-Pro-Pro-Ala-Lys(Boc)-Leu-Gln(Trt)-Pro-Arg(Pbf)-OKb)

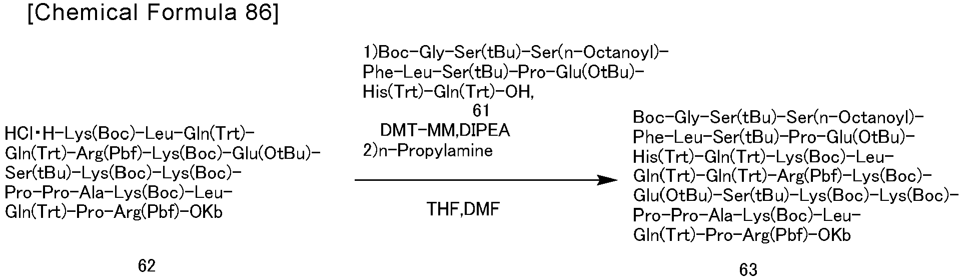

The compound 62 (251 mg, 0.1 mmol) was dissolved in a mixture liquid of THF (2 ml) and DMF (0.2 ml), the compound 61 (295 g, 0.15 mmol, 1.5 equiv) obtained in Example 37, DMT-MM (40.0 mg, 0.405 mmol, 1.45 equiv) and DIPEA (62 µL, 0.36 mmol, 3.6 equiv) were added and the mixture was stirred at room temperature for 30 minutes. As the amine scavenger, propylamine (25.0 µL, 0.3 mmol, 3 equiv) was added and the mixture was stirred at room temperature for 30 minutes. The reaction mixture was held under reduced pressure and the solvent was distilled off. To the residue was added cold acetonitrile and the deposited precipitate was filtrated, further, suspended and washed with cold acetonitrile, and the resultant solid was dried under reduced pressure, to obtain a compound 63 (562 mg, 84.4%).

### (Example 39) Synthesis of H-Gly-Ser-Ser(n-Octanoyl)-Phe-Leu-Ser-Pro-Glu-His-Gln-Lys-Leu-Gln-Gln-Arg-Lys-Glu-Ser-Lys-Lys-Pro-Pro-Ala-Lys-Leu-Gln-Pro-Arg-OH (compound 64)

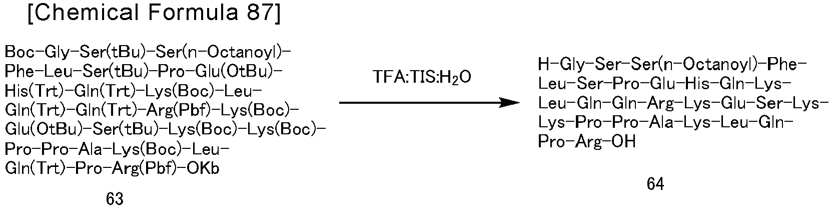

The compound 63 (555 mg, 0.083 mmol) obtained in Example 38 was dissolved in 8 ml of a mixture of TFA:TIS:water=95:2.5:2.5, and the mixture was stirred at room temperature for 3 hours. The reaction liquid was filtrated through Celite. The filtration residue was washed with 3 ml of TFA and filtrated. The resultant filtrates were combined together, and concentrated under reduced pressure at room temperature to 4.5 ml. The residue was put into 45 ml of cold isopropyl ether while stirring. The resultant solid was centrifugally separated, and the filtration residue was further suspended and washed with cold isopropyl ether and centrifugally separated, twice. The resultant solid was dried, to obtain a compound 64 (351 mg, Quant, HPLC purity 68.92%). The resultant compound was measured by LC-MS, to observe 1119.3 [M+3H⁺]/3.

### [Industrial Applicability]

When the peptide synthesis method of the present invention is used, double hit of an amino acid can be prevented in de-protection by deactivating an amino acid active species using a specific scavenger before de-protection of the N-terminal, even if the amino acid active species is not removed from the reaction system. The method of the present invention can solve the problem of an amino acid active species existing in the reaction system in the de-protection reaction, by a simple means, thus, this method is excellent in versatility and useful. Further, a peptide synthesized by the present invention shows less problems of deletion of an amino acid and double hit, and according to the present invention, a peptide of high quality can be synthesized at high yield.

## Claims

1. A peptide synthesis method comprising the following steps a to d:
a. condensing an amino acid having an N-terminal protected with fluorenylmethoxycarbonyl (N-Fmoc protected) or an N-Fmoc protected peptide with an amino acid having a C-terminal protected with a carrier, the carrier being capable to crystallize in response to a change of a composition of a dissolving solvent (C-carrier protected), a C-carrier protected peptide or a C-carrier protected amino acid amide, in the presence of a condensing agent, to obtain an N-Fmoc-C-carrier protected peptide,
b. subsequently adding to the reaction system resulting from step (a) at least one amine selected from the group consisting of an alkylamine having 1 to 14 carbon atoms, an aromatic amine having 1 to 14 carbon atoms and hydroxyl amine, wherein the alkylamine or the aromatic amine is a primary or secondary amine,
c. thereafter de-protecting the N-terminal, and
d. changing the composition of the solvent dissolving the resultant C-carrier protected peptide, to crystallize and separate the C-carrier protected peptide.

2. The peptide synthesis method according to Claim 1, wherein said carrier is a compound selected from the group consisting of compounds having the following structures: 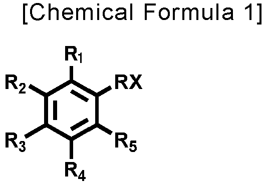 wherein R₁ and R₅ represent a hydrogen atom, R₂, R₃ and R₄ represent an alkoxyl group having 8 to 30 carbon atoms, and RX is a group represented by the following formula 2 and binding to the C-terminal of a peptide or an amino acid, 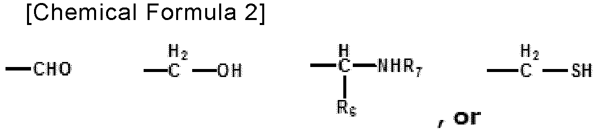 wherein R₇ represents a hydrogen atom, an alkyl group having 1 to 6 carbon atoms, a benzyl group or an alkoxy-substituted benzyl group, and R₆ represents a hydrogen atom, a phenyl group or an alkoxy-substituted phenyl group,
a compound having the following structure: 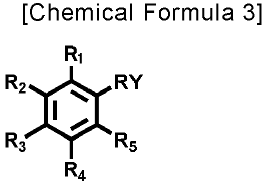 wherein R₂, R₄ and R₅ represent a hydrogen atom, R₁ and R₃ represent an alkoxyl group having 12 to 30 carbon atoms, and RY is a group represented by the following formula 4 and binding to the C-terminal of a peptide or an amino acid, 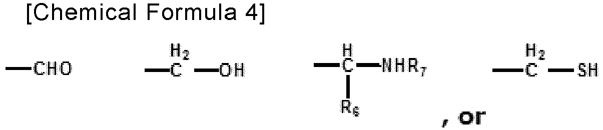 wherein R₇ represents a hydrogen atom, an alkyl group having 1 to 6 carbon atoms, a benzyl group or an alkoxy-substituted benzyl group, and R₆ represents a hydrogen atom, a phenyl group or an alkoxy-substituted phenyl group, an
d
a compound having the following structure: 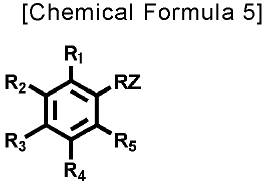 wherein R₁, R₃ and R₅ represent a hydrogen atom, R₂ and R₄ represent an alkoxyl group having 12 to 30 carbon atoms, and RZ is a group represented by the following formula 6 and binding to the C-terminal of a peptide or an amino acid, 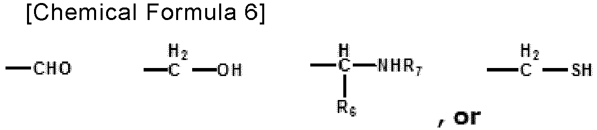 wherein R₇ represents a hydrogen atom, an alkyl group having 1 to 6 carbon atoms, a benzyl group or an alkoxy-substituted benzyl group, and R₆ represents a hydrogen atom, a phenyl group or an alkoxy-substituted phenyl group,
wherein the above formulae are shown in the state before binding to the C-terminal of a peptide or an amino acid.

3. The peptide synthesis method according to Claim 1, wherein said carrier is a compound selected from the group consisting of 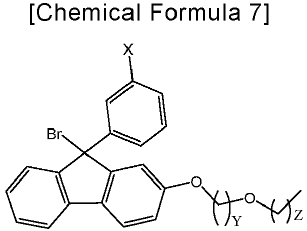 wherein X represents a halogen, Y is an integer of 8 to 12 and Z is an integer of 17 to 29, 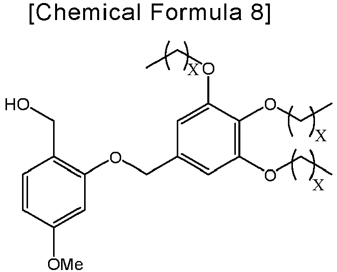 wherein X each independently represent an integer of 7 to 21, and 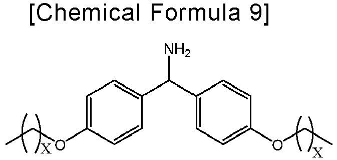 wherein X each independently represent an integer of 11 to 29,
wherein the above formulae are shown in the state before binding to the C-terminal of a peptide or an amino acid.

4. The peptide synthesis method according to Claim 1, wherein said carrier is a compound selected from the group consisting of 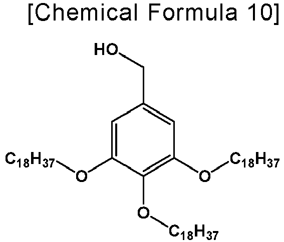 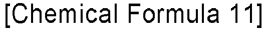 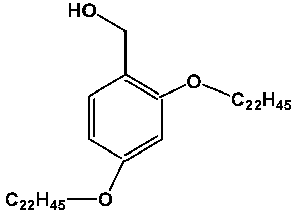 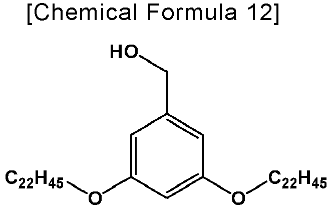 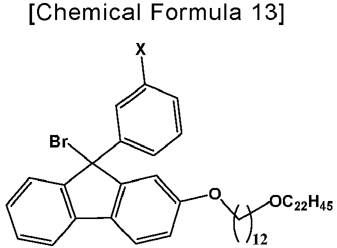 wherein X is F or CI 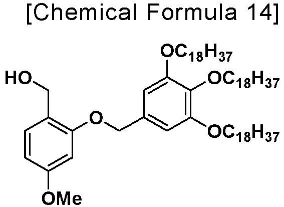 , and 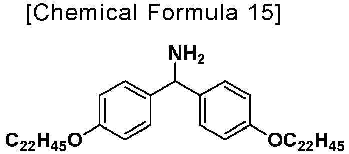 wherein the above formulae are shown in the state before binding to the C-terminal of a peptide or an amino acid.

5. The peptide synthesis method according to any one of Claims 1 to 4, wherein said amine is an alkylamine having 1 to 10 carbon atoms or hydroxylamine.

6. The peptide synthesis method according to any one of Claims 1 to 5, wherein said amine is an alkylamine having 3 or 4 carbon atoms.

7. The peptide synthesis method according to any one of Claims 1 to 6, wherein the amine equivalent in the step b is a 1 to 30-fold amount with respect to the amino acid equivalent theoretically remaining after the condensation reaction of the step a.

8. The peptide synthesis method according to any one of Claims 1 to 7, wherein the composition changing means for changing the composition of the solvent dissolving the resultant C-carrier protected peptide is performed by concentrating the solvent of the solution, then, adding a poor solvent to attain solidification.

9. The peptide synthesis method according to Claim 8, wherein said poor solvent is a solvent selected from the group consisting of acetonitrile, aqueous acetonitrile, methanol, aqueous methanol and water.

10. The peptide synthesis method according to any one of Claims 1 to 9, wherein in step b the at least one amine is added to scavenge active esters.

11. The peptide synthesis method according to any one of Claims 1 to 10, comprising conducting repetition of the step a to the step d using the C-carrier protected peptide crystallized and separated in the step d.

12. The peptide synthesis method according to any one of Claims 1 to 11, further comprising the following step:
e. a step of washing the crystal of the C-carrier protected peptide crystallized and separated with an organic solvent.

13. The peptide synthesis method according to any one of Claims 1 to 12, wherein said steps a to c are conducted in one pot synthesis.

## Patentansprüche

1. Ein Verfahren zur Peptidsynthese umfassend die folgenden Schritte a bis d:
a. Kondensierung einer Aminosäure mit einem N-Terminus, welcher mit Fluorenylmethoxycarbonyl (N-Fmoc-geschützt) geschützt ist, oder eines N-Fmocgeschützten Peptids mit einer Aminosäure mit einem C-Terminus, welcher mit einer Carrier geschützt ist, wobei der Carrier dazu in der Lage ist, als Reaktion auf eine Änderung der Zusammensetzung eines Lösemittels zu kristallisieren (C-Carriergeschützt), einen C-Carrier-geschützten Peptid oder einem C-Carrier-geschützten Aminosäureamid, in der Gegenwart eines Kondensierungsmittels, um ein N-Fmoc-C-Carrier-geschütztes Peptid zu erhalten,
b. anschließende Zugabe zum aus dem Schritt a resultierenden Reaktionssystem von wenigstens einem Amin ausgewählt aus der Gruppe bestehend aus einem Alkylamin mit 1 bis 14 Kohlenstoffatomen, einem aromatischen Amin mit 1 bis 14 Kohlenstoffatomen und Hydroxylamin, wobei das Alkylamin oder aromatische Amin ein primäres oder sekundäres Amin ist,
c. danach Entschützen des N-Terminus, und
d. Veränderung der Zusammensetzung des Lösemittels, welches das resultierende C-Carrier-geschützte Peptid löst, und das C-Carrier-geschützten Peptid zu kristallisieren und abzutrennen.

2. Das Verfahren zur Peptidsynthese gemäß Anspruch 1, wobei der Carrier eine Verbindung ist ausgewählt aus der Gruppe bestehend aus Verbindungen mit den folgenden Strukturen: 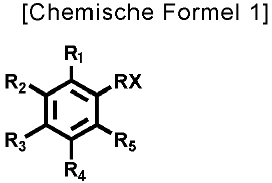 worin R₁ und R₅ ein Wasserstoffatom repräsentieren, R₂, R₃ und R₄ eine Alkoxylgruppe mit 8 bis 30 Kohlenstoffatomen repräsentieren, und RX eine Gruppe ist, welche durch die folgende Formel 2 repräsentiert wird und an den C-Terminus eines Peptids oder einer Aminosäure bindet, 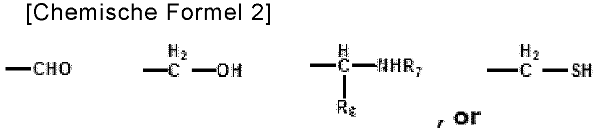 worin R₇ ein Wasserstoffatom, eine Alkylgruppe mit 1 bis 6 Kohlenstoffatomen, eine Benzylgruppe oder eine Alkoxy-substituierte Benzylgruppe repräsentiert, und R₆ ein Wasserstoffatom, eine Phenylgruppe oder eine Alkoxy-substituierte Phenylgruppe repräsentiert,
eine Verbindung mit der folgenden Struktur: 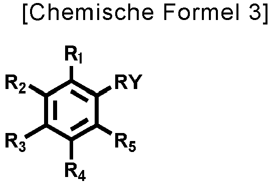 worin R₂, R₄ und R₅ ein Wasserstoffatom repräsentieren, R₁ und R₃ eine Alkoxylgruppe mit 12 bis 30 Kohlenstoffatomen repräsentieren, und RY eine Gruppe ist, welche durch die folgende Formel 4 repräsentiert wird und an den C-Terminus eines Peptids oder einer Aminosäure bindet, 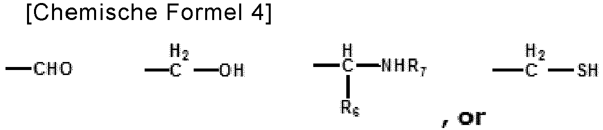 worin R₇ ein Wasserstoffatom, eine Alkylgruppe mit 1 bis 6 Kohlenstoffatomen, eine Benzylgruppe oder eine Alkoxy-substituierte Benzylgruppe repräsentiert, und R₆ ein Wasserstoffatom, eine Phenylgruppe oder eine Alkoxy-substituierte Phenylgruppe repräsentiert,
und
eine Verbindung mit der folgenden Struktur: 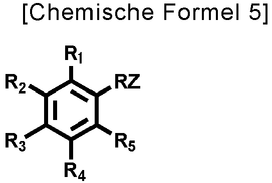 worin R₁ , R₃ und R₅ ein Wasserstoffatom repräsentieren, R₂ und R₄ eine Alkoxylgruppe mit 12 bis 30 Kohlenstoffatomen repräsentieren, und RZ eine Gruppe ist, welche durch die folgende Formel 6 repräsentiert wird und an den C-Terminus eines Peptids oder einer Aminosäure bindet, 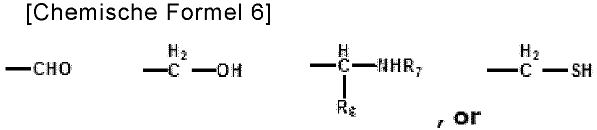 worin R₇ ein Wasserstoffatom, eine Alkylgruppe mit 1 bis 6 Kohlenstoffatomen, eine Benzylgruppe oder eine Alkoxy-substituierte Benzylgruppe repräsentiert, und R₆ ein Wasserstoffatom, eine Phenylgruppe oder eine Alkoxy-substituierte Phenylgruppe repräsentiert,
wobei die obigen Formeln gezeigt werden in dem Zustand, in dem sie sind bevor sie an den C-Terminus eines Peptids oder einer Aminosäure binden.

3. Das Verfahren zur Peptidsynthese gemäß Anspruch 1, wobei der Carrier eine Verbindung ist ausgewählt aus der Gruppe bestehend aus 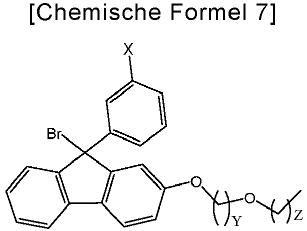 worin X ein Halogen repräsentiert, Y eine ganze Zahl von 8 bis 12 ist, und Z eine ganze Zahl von 17 bis 29 ist, 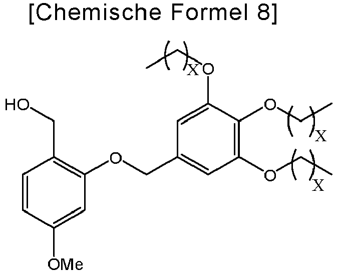 worin jedes X unabhängig von den anderen eine ganze Zahl von 7 bis 21 repräsentiert, und 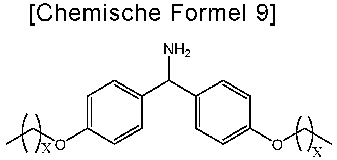 worin jedes X unabhängig von den anderen eine ganze Zahl von 11 bis 29 repräsentiert,
wobei die obigen Formeln gezeigt werden in dem Zustand, in dem sie sind bevor sie an den C-Terminus eines Peptids oder einer Aminosäure binden.

4. Das Verfahren zur Peptidsynthese gemäß Anspruch 1, wobei der Carrier eine Verbindung ist ausgewählt aus der Gruppe bestehend aus 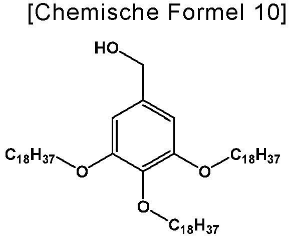 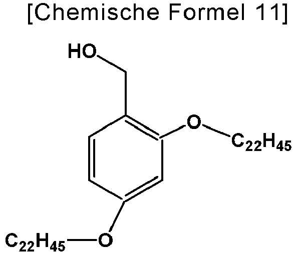 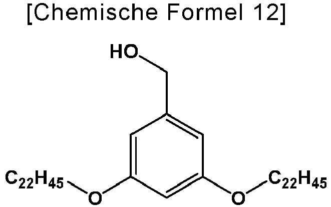 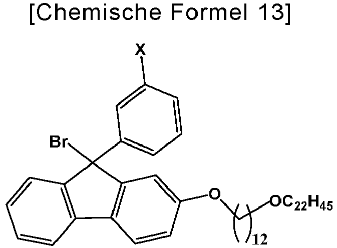 worin X F oder Cl ist 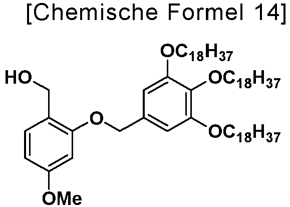 , und 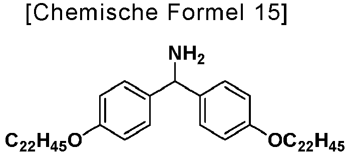 wobei die obigen Formeln gezeigt werden in dem Zustand, in dem sie sind bevor sie an den C-Terminus eines Peptids oder einer Aminosäure binden.

5. Das Verfahren zur Peptidsynthese gemäß irgendeinem der Ansprüche 1 bis 4, wobei das Amin ein Alkylamin mit 1 bis 10 Kohlenstoffatomen oder Hydroxylamin ist.

6. Das Verfahren zur Peptidsynthese gemäß irgendeinem der Ansprüche 1 bis 5, wobei das Amin ein Alkylamin mit 3 oder 4 Kohlenstoffatomen ist.

7. Das Verfahren zur Peptidsynthese gemäß irgendeinem der Ansprüche 1 bis 6, wobei das Aminäquivalent im Schritt b die 1- bis 30-fache Menge bezogen auf das Aminosäureäquivalent ist, welches theoretisch nach der Kondensationsreaktion von Schritt a übrigbleibt.

8. Das Verfahren zur Peptidsynthese gemäß irgendeinem der Ansprüche 1 bis 7, wobei die Maßnahme zur Veränderung der Zusammensetzung des Lösemittels, in welchem das resultierende C-Carrier-geschützte Peptid gelöst ist, durchgeführt wird mittels Konzentrieren des Lösemittels der Lösung und anschließender Zugabe eines schlechten Lösemittels, um das Ausfällen zu bewirken.

9. Das Verfahren zur Peptidsynthese gemäß Anspruch 8, wobei das schlechte Lösemittel ein Lösemittel ist ausgewählt aus der Gruppe bestehend aus Acetonitril, wässrigem Acetonitril, Methanol, wässrigem Methanol, und Wasser.

10. Das Verfahren zur Peptidsynthese gemäß irgendeinem der Ansprüche 1 bis 9, wobei in Schritt b wenigstens ein Amin hinzugefügt wird, um aktive Ester abzufangen.

11. Das Verfahren zur Peptidsynthese gemäß irgendeinem der Ansprüche 1 bis 10, umfassend die Wiederholung von Schritt a bis Schritt d unter Verwendung des in Schritt d kristallisierten und abgetrennten C-Carrier-geschützten Peptids.

12. Das Verfahren zur Peptidsynthese gemäß irgendeinem der Ansprüche 1 bis 11, des Weiteren umfassend den folgenden Schritt:
e. einen Schritt, bei dem der Kristall des C-Carrier-geschützten Peptids, das kristallisiert und abgetrennt wurde, mit einem organischen Lösemittel gewaschen wird.

13. Das Verfahren zur Peptidsynthese gemäß irgendeinem der Ansprüche 1 bis 12, wobei die Schritte a bis c als Eintopf-Synthese durchgeführt werden.

## Revendications

1. Procédé de synthèse peptidique comprenant les étapes a à d suivantes :
a. la condensation d'un acide aminé ayant une extrémité N-terminale protégée par fluorénylméthoxycarbonyle (protégée par N-Fmoc) ou d'un peptide protégé par N-Fmoc avec un acide aminé ayant une extrémité C-terminale protégée par un support, le support étant capable de cristalliser en réponse à un changement de composition d'un solvant de dissolution (protégé par C-porteur), d'un peptide protégé par C-porteur ou d'un amide d'acide aminé protégé par C-porteur, en présence d'un agent de condensation, pour obtenir un peptide protégé par N-Fmoc-C-porteur,
b. l'ajout subséquent au système réactionnel résultant de l'étape (a) d'au moins une amine choisie dans le groupe constitué par une alkylamine ayant 1 à 14 atomes de carbone, une amine aromatique ayant 1 à 14 atomes de carbone et une hydroxylamine, où l'alkylamine ou l'amine aromatique est une amine primaire ou secondaire,
c. ensuite, la déprotection de l'extrémité N-terminale, et
d. la modification de la composition du solvant dissolvant le peptide protégé par C-porteur résultant, pour cristalliser et séparer le peptide protégé par C-porteur.

2. Procédé de synthèse peptidique selon la revendication 1, où ledit support est un composé choisi dans le groupe constitué par des composés ayant les structures suivantes : 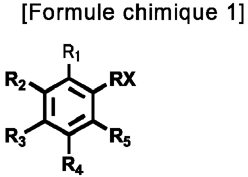 où R₁ et R₅ représentent un atome d'hydrogène, R₂, R₃ et R₄ représentent un groupe alcoxyle ayant 8 à 30 atomes de carbone, et RX est un groupe représenté par la formule 2 suivante et se liant à l'extrémité C-terminale d'un peptide ou d'un acide aminé, 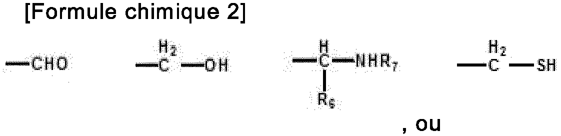 où R₇ représente un atome d'hydrogène, un groupe alkyle ayant 1 à 6 atomes de carbone, un groupe benzyle ou un groupe benzyle substitué par alcoxy, et R₆ représente un atome d'hydrogène, un groupe phényle ou un groupe phényle substitué par alcoxy,
un composé ayant la structure suivante : 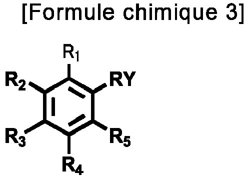 où R₂, R₄ et R₅ représentent un atome d'hydrogène, R₁ et R₃ représentent un groupe alcoxyle ayant 12 à 30 atomes de carbone, et RY est un groupe représenté par la formule 4 suivante et se liant à l'extrémité C-terminale d'un peptide ou d'un acide aminé, 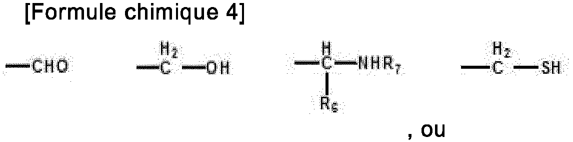 où R₇ représente un atome d'hydrogène, un groupe alkyle ayant 1 à 6 atomes de carbone, un groupe benzyle ou un groupe benzyle substitué par alcoxy, et R₆ représente un atome d'hydrogène, un groupe phényle ou un groupe phényle substitué par alcoxy,
et
un composé ayant la structure suivante : 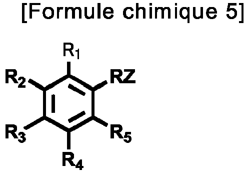 où R₁, R₃ et R₅ représentent un atome d'hydrogène, R₂ et R₄ représentent un groupe alcoxyle ayant 12 à 30 atomes de carbone, et RZ est un groupe représenté par la formule 6 suivante et se liant à l'extrémité C-terminale d'un peptide ou d'un acide aminé, 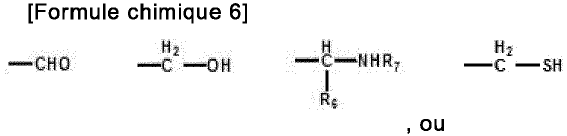 où R₇ représente un atome d'hydrogène, un groupe alkyle ayant 1 à 6 atomes de carbone, un groupe benzyle ou un groupe benzyle substitué par alcoxy, et R₆ représente un atome d'hydrogène, un groupe phényle ou un groupe phényle substitué par alcoxy,
où les formules ci-dessus sont présentées dans l'état précédant la liaison à l'extrémité C-terminale d'un peptide ou d'un acide aminé.

3. Procédé de synthèse peptidique selon la revendication 1, où ledit support est un composé choisi dans le groupe constitué par 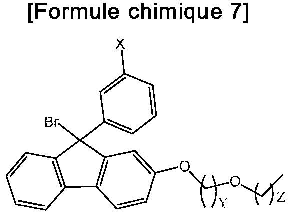 où X représente un halogène, Y est un nombre entier de 8 à 12 et Z est un nombre entier de 17 à 29, 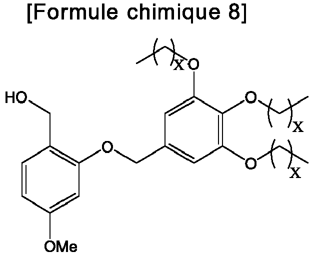 où chaque X représente indépendamment un nombre entier de 7 à 21, et 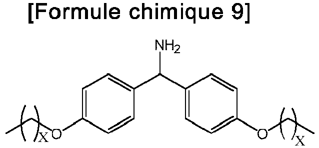 où chaque X représente indépendamment un nombre entier de 11 à 29,
où les formules ci-dessus sont présentées dans l'état précédant la liaison à l'extrémité C-terminale d'un peptide ou d'un acide aminé.

4. Procédé de synthèse peptidique selon la revendication 1, où ledit support est un composé choisi dans le groupe constitué par 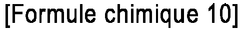 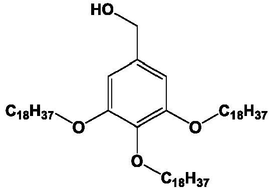 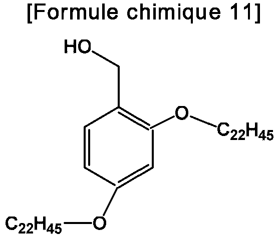 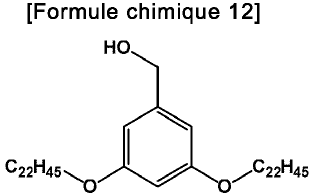 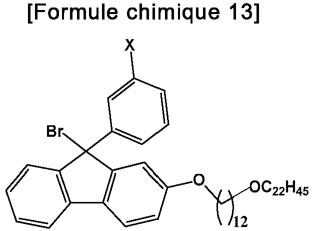 où X est F ou Cl 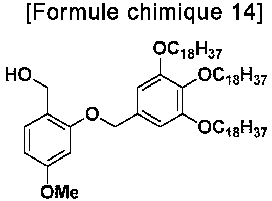 , et 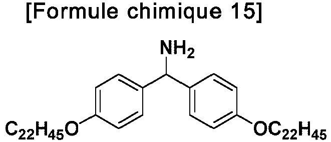 où les formules ci-dessus sont présentées dans l'état précédant la liaison à l'extrémité C-terminale d'un peptide ou d'un acide aminé.

5. Procédé de synthèse peptidique selon l'une quelconque des revendications 1 à 4, où ladite amine est une alkylamine ayant 1 à 10 atomes de carbone ou une hydroxylamine.

6. Procédé de synthèse peptidique selon l'une quelconque des revendications 1 à 5, où ladite amine est une alkylamine ayant 3 ou 4 atomes de carbone.

7. Procédé de synthèse peptidique selon l'une quelconque des revendications 1 à 6, où l'équivalent amine à l'étape b est une quantité de 1 à 30 fois supérieure à l'équivalent acide aminé restant théoriquement après la réaction de condensation de l'étape a.

8. Procédé de synthèse peptidique selon l'une quelconque des revendications 1 à 7, où le moyen de modification de composition pour la modification de la composition du solvant dissolvant le peptide résultant protégé par C-porteur est réalisé par concentration du solvant de la solution, puis par addition d'un solvant pauvre pour obtenir une solidification.

9. Procédé de synthèse peptidique selon la revendication 8, où ledit solvant pauvre est un solvant choisi dans le groupe constitué par l'acétonitrile, l'acétonitrile aqueux, le méthanol, le méthanol aqueux et l'eau.

10. Procédé de synthèse peptidique selon l'une quelconque des revendications 1 à 9, où, à l'étape b, au moins une amine est ajoutée pour piéger des esters actifs.

11. Procédé de synthèse peptidique selon l'une quelconque des revendications 1 à 10, comprenant le fait de conduire une répétition de l'étape a à l'étape d en utilisant le peptide protégé par C-porteur cristallisé et séparé à l'étape d.

12. Procédé de synthèse peptidique selon l'une quelconque des revendications 1 à 11, comprenant en outre l'étape suivante :
e. une étape de lavage du cristal du peptide protégé par C-porteur cristallisé et séparé avec un solvant organique.

13. Procédé de synthèse peptidique selon l'une quelconque des revendications 1 à 12, où lesdites étapes a à c sont conduites dans une synthèse monotope.
